# EUROPEAN PATENT SPECIFICATION

(11) **EP 1 790 659 B1**
(45) Date of publication and mention of the grant of the patent: **31.07.2013**
(21) Application number: 06022429.2
(22) Date of filing: 13.11.2002
(51) Int. Cl.: C07K 14/21, A61K 39/104, C12N 15/62, G01N 33/53

(54) **Polypeptides of pseudomonas aeruginosa**
Pseudomonas aeruginosa Polypeptide
Polypeptides de pseudomonas aeruginosa

(30) Priority: 13.11.2001 US 331221 P
(43) Date of publication of application: 30.05.2007
(62) Divisional of application: 02774222.0
(73) Proprietor: ID Biomedical Corporation, Laval, QC H7V 3S8 (CA)
(72) Inventor: Charland, Nathalie, Breakeyville, Quebec G0S 1E3 (CA); Hamel, Josee, Sillery, Quebec G1T 1N6 (CA); Brodeur, Bernard, R., Sillery, Quebec G1T 1N6 (CA); Martin, Denis, St-Augustin-de-Desmaures Quebec G3A 1E9 (CA); Charlebois, Isabelle, St-Nicolas, Quebec G7A 2L5 (CA); Bussiere, Diane, St-Nicolas, Quebec, G7A 1W9 (CA)
(74) Representative: Johnston, Caroline Louise

(56) References cited:
- EP-A- 0 297 291
- EP-A- 0 717 106
- WO-A-01/02577
- WO-A-01/40473
- WO-A-99/57142
- WO-A-02/064161
- STOVER C K ET AL: "COMPLETE GENOME SEQUENCE OF PSEUDOMONAS AERUGINOSA PA01, AN OPPORTUNISTIC PATHOGEN" NATURE, MACMILLAN JOURNALS LTD. LONDON, GB, [Online] vol. 406, no. 6799, 31 August 2000 (2000-08-31), pages 959-964, XP000996980 ISSN: 0028-0836 -& DATABASE EMBL [Online] 1 March 2001 (2001-03-01), STOVER ET AL: XP002267590 retrieved from EBI Database accession no. Q9HX28 -& DATABASE EMBL [Online] 1 September 2000 (2000-09-01), STOVER ET AL.: XP002267591 retrieved from EBI Database accession no. AE004817
- DATABASE UNIPROT [Online] 01 March 2001 'SubName: Full=Putative uncharacterized protein;' Retrieved from EBI, accession no. UNIPROT:Q9HX32 Database accession no. Q9HX32

## Description

### FIELD OF THE INTENTION

The present invention is related to polypeptides, more particularly SPA-2 polypeptides of Pseudomonas aeruginosa which may be used to prevent, diagnose and/or treat Pseudomonas aeruginosa infection.

### BACKGROUND OF THE INVENTION

Pseudomonas aeruginosa is a prevalent opportunistic bacterial pathogen in humans and animals. P. aeruginosa is the most common Gram-negative bacterium found in nosocomial infections, especially in immunocompromised individuals. It is frequently related to ventilator-associated pneumonia in intubated patients. Pseudomonas infection is common amongst patients with cystic fibrosis, burn wounds, organ transplants, and intravenous-drug addiction. Cystic fibrosis patients are often chronically infected by P. aeruginosa, which is responsible for increased illness and death in this particular population. P. aeruginosa bacteremia is responsible for high death rates in burn units. Pseudomonas can lead to serious conditions such as endophthalmitis, endocarditis, meningitis, pneumonia, and septicaemia. Septicemia due to P. aeruginosa is associated with the highest death rates of all Gram-negative infections.

Since P. aeruginosa is naturally resistant to many antibiotics, there is a need for the development of a vaccine that will protect individuals from P. aeruginosa infection. An infection by P. aeruginosa induces an immune response against antigens found at the surface of the bacterial cells. However, many of these surface proteins are still not characterized, nor has the immune response resulting in protection from infection by different strains been determined.

To develop a vaccine that will protect individuals from P. aeruginosa infection, efforts have mainly been concentrated on lipopolysaccharides (LPS). However, even though a limited number of LPS serotypes are associated with clinical cases, the production of a multivalent LPS-based vaccine is complex and may induce serotype replacement in vaccinated individuals. Anti-flagellar and anti-pili vaccines are also evaluated but the regulation of flagella/pili expression at different P. aeruginosa infection stages may prevent effective protection. Outer membrane proteins (OMP) are also being tested. An OMP preparation from 4 different P. aeruginosa serotypes is currently in clinical trials but the specificity of the protection confered by this preparation remains to be evaluated. A recombinant fusion protein, based on outer membrane proteins OprF and OprI, is considered a promising vaccine candidate. However, the OprF protein was shown to be absent from some clinical strains of P. aeruginosa and the protection confered by the OprI protein alone has not been evaluated yet.

A review of existing technology is described in Stanislavsky ES and Lam JS. (1997) FEMS Microbiol. Rev. 21(3): 243-77 and Holder IA. (2001) J. Burn Care Rehabil. 22(5): 311-20.

The sequence of the genome of P. aeruginosa strain PAO1 was determined in a collaboration among the Cystic Fibrosis Foundation, the University of Washington and Pathogenesis Corporation and is available at http://www.pseudomonas.com/, http://www.tigr.org/tigr-scripts/CMR2/GenomePage3.spl?database=n tpa03, http://pseudomonas.bit.uq.edu.au/ and in Nature, Stover et al. 406:959-964 (2000).

WO 99/57142, EP0717106, EP0297291 and WO 01/40473 disclose *P. aeruginosa* polypeptides.

Therefore there remains an unmet need for P. aeruginosa polypeptides that may be used to prevent, diagnose and/or treat P. aeruginosa infection.

### SUMMARY OF THE INVENTION

According to one aspect, the present invention relates to claim 1

### BRIEF DESCRIPTION OF THE DRAWINGS

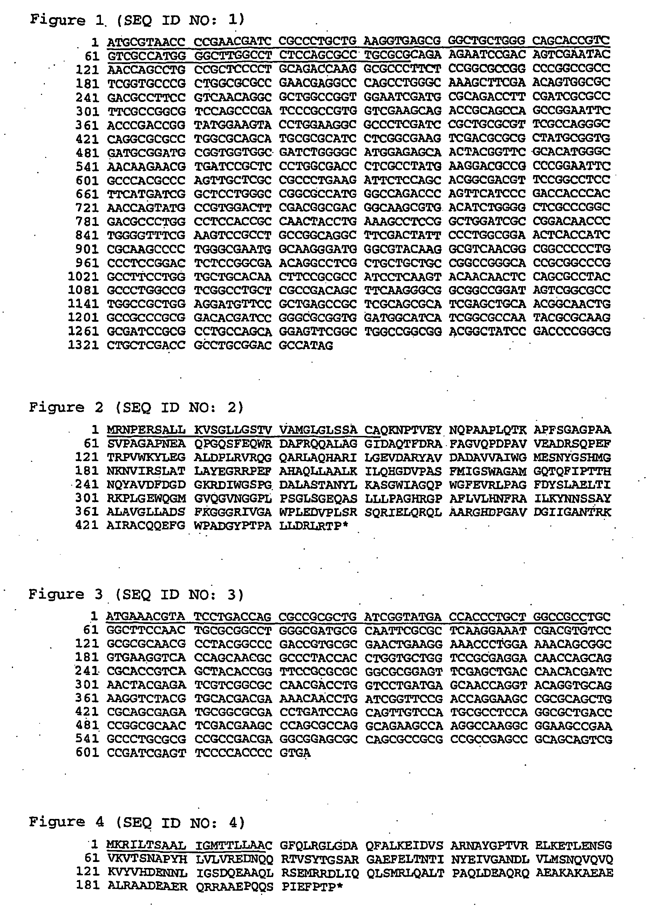

Figure 1 represents the DNA sequence of SPA-1 gene from P. aeruginosa strain PAO1; SEQ ID NO: 1. The underlined portion of the sequence represents the leader peptide coding region.
Figure 2 represents the amino acid sequence of SPA-1 polypeptide from P. aeruginosa strain PAO1; SEQ ID NO: 2. The underlined sequence represents the 32 amino acid residues leader peptide.
Figure 3 represents the DNA sequence of SPA-2 gene from P. aeruginosa strain PAO1; SEQ ID NO: 3. The underlined portion of the sequence represents the leader peptide coding region.
Figure 4 represents the amino acid sequence of SPA-2 polypeptide from P. aeruginosa strain PAO1; SEQ ID NO: 4. The underlined sequence represents the 19 amino acid residues leader peptide.
Figure 5 represents the DNA sequence of SPA-3 gene from P. aeruginosa strain PAO1; SEQ ID NO: 5. The underlined portion of the sequence represents the leader peptide coding region.
Figure 6 represents the amino acid sequence of SPA-3 polypeptide from P. aeruginosa strain PAO1; SEQ ID NO: 6. The underlined sequence represents the 21 amino acid residues leader peptide.
Figure 7 represents the DNA sequence of SHB-PA104 gene from P. aeruginosa strain PAO1; SEQ ID NO: 19. The underlined portion of the sequence represents the leader peptide-coding region.
Figure 8 represents the amino acid sequence of SHB-PA104 protein from P. aeruginosa strain PAO1;. SEQ ID NO: 20. The underlined sequence represents the 16 amino acid residues leader peptide. Figure 9 represents the DNA sequence of SHB-PA105 gene from P. aeruginosa strain PAO1; SEQ ID NO: 21. The underlined portion of the sequence represents the leader peptide-coding region.
Figure 10 represents the amino acid sequence of SHB-PA105 protein from P. aeruginosa strain PAO1; SEQ ID NO: 22. The underlined sequence represents the 33 amino acid residues leader peptide.
Figure 11 represents the DNA sequence of SHB-PA106 gene from P. aeruginosa strain PAO1; SEQ ID NO: 23. The underlined portion of the sequence represents the leader peptide-coding region.
Figure 12 represents the amino acid sequence of SHB-PA106 protein from P. aeruginosa strain PAO1; SEQ ID NO: 24. The underlined sequence represents the 16 amino acid residues leader peptide.
Figure 13 represents the protein sequence alignment of SPA-1 protein with SHB-PA104 (without leader peptides) from PAO1 strain. |, identical amino acids; :, conserved amino acids.
Figure 14 represents the protein sequence alignment of SPA-1 protein with SHB-PA105 (without leader peptides) from PAO1 strain. |, identical amino acids; :, conserved amino acids.
Figure 15 represents the protein sequence alignment of SPA-1 protein with SHB-PA106 (without leader peptides) from PAO1 strain. |, identical amino acids; :, conserved amino acids.

### DETAILED DESCRIPTION OF THE INVENTION

The present invention provides purified and isolated polynucleotides, which encode Pseudomonas polypeptides which may be used to prevent, diagnose and/or treat Pseudomonas infection.

According to one aspect, the present invention provides an isolated polynucleotide encoding a polypeptide having at least 90% identity to a second polypeptide comprising a sequence chosen from SEQ ID NO: 4 or fragments or analogs thereof.

According to one aspect, the present invention provides an isolated polynucleotide encoding a polypeptide having at least 95% identity to a second polypeptide comprising a sequence chosen from SEQ ID NO: 4 or fragments or analogs thereof.

According to one aspect, the present invention provides an isolated polynucleotide encoding a polypeptide having at least 98% identity to a second polypeptide comprising a sequence chosen from SEQ ID NO: 4 or fragments or analogs thereof.

According to one aspect, the present invention provides an isolated polynucleotide encoding a polypeptide having at least 90% identity to a second polypeptide comprising SEQ ID NO: 4.

According to one aspect, the present invention provides an isolated polynucleotide encoding a polypeptide having at least 95% identity to a second polypeptide comprising SEQ ID NO: 4.

According to one aspect, the present invention provides an isolated polynucleotide encoding a polypeptide having at least 98% identity to a second polypeptide comprising SEQ ID NO: 4.

According to one aspect, the present invention relates to polypeptides which comprise an amino acid sequence selected from SEQ ID NO: 4 or fragments or analogs thereof.

According to one aspect, the present invention relates to polypeptides which comprise an amino acid sequence selected from SEQ ID NO: 4.

According to one aspect, the present invention relates to polypeptides characterized by the amino acid sequence comprising SEQ ID NO: 4 or fragments or analogs thereof.

According to one aspect, the present invention relates to polypeptides characterized by the amino acid sequence comprising SEQ ID NO: 4.

According to one aspect, the present invention provides a polynucleotide encoding an epitope bearing portion of a polypeptide comprising a sequence chosen from SEQ ID NO: 4 or fragments or analogs thereof.

According to one aspect, the present invention provides a polynucleotide encoding an epitope bearing portion of a polypeptide comprising a sequence chosen from SEQ ID NO: 4.

According to one aspect, the present invention relates to epitope bearing portions of a polypeptide comprising a sequence chosen from SEQ ID NO: 4 or fragments or analogs thereof.

According to one aspect, the present invention relates to epitope bearing portions of a polypeptide comprising a sequence chosen from SEQ ID NO: 4.

According to one aspect, the present invention provides an isolated polynucleotide comprising a polynucleotide chosen from:
(a) a polynucleotide encoding a polypeptide having at least 90% identity to a second polypeptide comprising a sequence chosen from: SEQ ID NO: 4 or fragments or analogs thereof;
(c) a polynucleotide encoding a polypeptide having at least 95% identity to a second polypeptide comprising a sequence chosen from: SEQ ID NOS: 2, 4, 6, 8, 10, 12 or fragments or analogs thereof;
(d) a polynucleotide encoding a polypeptide comprising a sequence chosen from: SEQ ID NO: 4 or fragments or analogs thereof;
(e) a polynucleotide encoding a polypeptide capable of raising antibodies having binding specificity for a polypeptide comprising a sequence chosen from: *SEQ ID NO*: 4 or fragments or analogs thereof;
(f) a polynucleotide encoding an epitope bearing portion of a polypeptide comprising a sequence chosen from SEQ ID NO: 4 or fragments or analogs thereof;
(g) a polynucleotide comprising a sequence chosen from SEQ ID NO: 3 or fragments or analogs thereof;
(h) a polynucleotide that is complementary to a polynucleotide in (a), (b), (c), (d), (e), (f) or (g).

According to one aspect, the present invention provides an isolated polynucleotide comprising a polynucleotide chosen from:
(a) a polynucleotide encoding a polypeptide having at least 90% identity to a second polypeptide comprising a sequence chosen from: SEQ ID NO: 4;
(c) a polynucleotide encoding a polypeptide having at least 95% identity to a second polypeptide comprising a sequence chosen from: SEQ ID NO: 4;
(d) a polynucleotide encoding a polypeptide comprising a sequence chosen from: SEQ ID NO: 4;
(e) a polynucleotide encoding a polypeptide capable of raising antibodies having binding specificity for a polypeptide comprising a sequence chosen from: SEQ ID NO: 4;
(f) a polynucleotide encoding an epitope bearing portion of a polypeptide comprising a sequence chosen from SEQ ID NO: 4;
(g) a polynucleotide comprising a sequence chosen from SEQ ID NO: 3;
(h) a polynucleotide that is complementary to a polynucleotide in (a), (b), (c), (d), (e), (f) or (g).

According to one aspect, the present invention provides an isolated polypeptide comprising a polypeptide chosen from:
(a) a polypeptide having at least 90% identity to a second polypeptide comprising a sequence chosen from SEQ ID NO: 4 or fragments or analogs thereof;
(c) a polypeptide having at least 95% identity to a second polypeptide comprising a sequence chosen from SEQ ID NO: 4 or fragments or analogs thereof;
(d) a polypeptide comprising a sequence chosen from SEQ ID NO: 4 or fragments or analogs thereof;
(e) a polypeptide capable of raising antibodies having binding specificity for a polypeptide comprising a sequence chosen from SEQ ID NO: 4 or fragments or analogs thereof;
(f) an epitope bearing portion of a polypeptide comprising a sequence chosen from SEQ ID NO: 4 or fragments or analogs thereof;
(g) the polypeptide of (a), (b), (c), (d), (e) or (f) wherein the N-terminal Met residue is deleted;
(h) the polypeptide of (a), (b), (c), (d), (e), (f) or (g) wherein the secretory amino acid sequence is deleted.

According to one aspect, the present invention provides an isolated polypeptide comprising a polyeptide chosen from:
(a) a polypeptide having at least 90% identity to a second polypeptide comprising a sequence chosen from SEQ ID NO:
(c) a polypeptide having at least 95% identity to a second polypeptide comprising a sequence chosen from SEQ ID NO: 4;
(d) a polypeptide comprising a sequence chosen from SEQ ID NO: 4;
(e) a polypeptide capable of raising antibodies having binding specificity for a polypeptide comprising a sequence chosen from SEQ ID NO: 4;
(f) an epitope bearing portion of a polypeptide comprising a sequence chosen from SEQ ID NO: 4;
(g) the polypeptide of (a), (b), (c), (d), (e) or (f) wherein the N-terminal Met residue is deleted;
(h) the polypeptide of (a), (b), (c), (d), (e), (f) or (g) wherein the secretory amino acid sequence is deleted.

Those skilled in the art will appreciate that the invention includes DNA molecules, i.e. polynucleotides, their homologous sequences and their complementary sequences that encode analogs such as mutants, variants, homologs and derivatives of such polypeptides, as described herein in the present patent application. Homologous genes are evolutionary related, have similar sequences and are structurally related. The invention also includes RNA molecules corresponding to the DNA molecules of the invention. In addition to the DNA and RNA molecules, the invention includes the corresponding polypeptides and monospecific antibodies that specifically bind to such polypeptides.

In a further embodiment, the polypeptides in accordance with the present invention are antigenic.

In a further embodiment, the polypeptides in accordance with the present invention are immunogenic.

In a further embodiment, the polypeptides in accordance with the present invention can elicit an immune response in a host.

In a further embodiment, the present invention also relates to polypeptides which are able to raise antibodies having binding specificity to the polypeptides of the present invention as defined above.

An antibody that "has binding specificity" is an antibody that recognizes and binds the selected polypeptide but which does not substantially recognize and bind other molecules in a sample, e.g., a biological sample. Specific binding can be measured using an ELISA assay in which the selected polypeptide is used as an antigen.

In accordance with the present invention, "protection" in the biological studies is defined by a significant increase in the survival curve, rate or period. Statistical analysis using the Log rank test to compare survival curves, and Fisher exact test to compare survival rates and numbers of days to death, respectively, might be useful to calculate P values and determine whether the difference between the two groups is statistically significant. P values of 0.05 are regarded as not significant.

In an additional aspect of the invention there are provided antigenic/immunogenic fragments of the polypeptides of the invention, or of analogs thereof.

The fragments of the present invention should include one or more such epitopic regions or be sufficiently similar to such regions to retain their antigenic/immunogenic properties. Thus, for fragments according to the present invention the degree of identity is perhaps irrelevant, since they may be 100% identical to a particular part of a polypeptide or analog thereof as described herein. The present invention further provides fragments having at least 20 contiguous amino acid residues from the polypeptide sequences of the present invention.

The skilled person will appreciate that analogs of the polypeptides of the invention will also find use in the context of the present invention, i.e. as antigenic/immunogenic material. Thus, for instance proteins or polypeptides which include one or more additions, deletions, substitutions or the like are encompassed by the present invention.

As used herein, "fragments", "analogs" or "derivatives" of the polypeptides of the invention include those polypeptides in which one or more of the amino acid residues are substituted with a conserved or non-conserved amino acid residue (preferably conserved) and which may be natural or unnatural. In one embodiment, derivatives and analogs of polypeptides of the invention will have greater than 90% identity with those sequences illustrated in the figures or fragments thereof. That is, 90% of the residues are the same.

In a further embodiment, polypeptides will have greater than 95% identity. In a further embodiment, polypeptides will have greater than 99% identity. In a further embodiment, analogs of polypeptides of the invention will have fewer than about 20 amino acid residue substitutions, modifications or deletions and more preferably less than 10.

In a further embodiment, polypeptides will have greater than 90% homology. In a further embodiment, polypeptides will have greater than 95% homology. In a further embodiment, polypeptides will have greater than 99% homology. In a further embodiment, derivatives and analogs of polypeptides of the invention will have less than about 20 amino acid residue substitutions, modifications or deletions and more preferably less than 10. Preferred substitutions are those known in the art as conserved i.e. the substituted residues share physical or chemical properties such as hydrophobicity, size, charge or functional groups.

These substitutions are those having a minimal influence on the secondary structure and hydropathic nature of the polypeptide. Preferred substitutions are those known in the art as conserved, i.e. the substituted residues share physical or chemical properties such as hydrophobicity, size, charge or functional groups. These include substitutions such as those described by Dayhoff, M. in Atlas of Protein Sequence and Structure 5, 1978 and by Argos, P. in EMBO J. 8, 779-785, 1989. For example, amino acids, either natural or unnatural, belonging to one of the following groups represent conservative changes:
ala, pro, gly, gln, asn, ser, thr, val;
cys, ser, tyr, thr;
val, ile, leu, met, ala, phe;
lys, arg, orn, his;
and phe, tyr, trp, his.

The preferred substitutions also include substitutions of D-enantiomers for the corresponding L-amino acids.

In an alternative approach, the analogs could be fusion polypeptides, incorporating moieties which render purification easier, for example by effectively tagging the desired polypeptide. It may be necessary to remove the "tag" or it may be the case that the fusion polypeptide itself retains sufficient antigenicity to be useful.

The percentage of homology is defined as the sum of the percentage of identity plus the percentage of similarity or conservation of amino acid type.

In one embodiment, analogs of polypeptides of the invention will have about 90% homology with those sequences illustrated in the figures or fragments thereof. In a further embodiment, polypeptides will have greater than 95% homology. In a further embodiment, polypeptides will have greater than 99% homology. In a further embodiment, analogs of polypeptides of the invention will have fewer than about 20 amino acid residue substitutions, modifications or deletions and more preferably less than 10.

One can use a program such as the CLUSTAL program to compare amino acid sequences. This program compares amino acid sequences and finds the optimal alignment by inserting spaces in either sequence as appropriate. It is possible to calculate amino acid identity or homology for an optimal alignment. A program like BLASTx will align the longest stretch of similar sequences and assign a value to the fit. It is thus possible to obtain a comparison where several regions of similarity are found, each having a different score. Both types of identity analysis are contemplated in the present invention.

In an alternative approach, the analogs or derivatives could be fusion polypeptides, incorporating moieties which render purification easier, for example by effectively tagging the desired protein or polypeptide, it may be necessary to remove the "tang", or it may be the case that the fusion polypeptide itself retains sufficient antigenicity to be useful.

It is well known that it is possible to screen an antigenic polypeptide to identify epitopic regions, i.e. those regions which are responsible for the polypeptide's antigenicity or immunogenicity. Methods for carrying out such screening are well known in the art. Thus, the fragments of the present invention should include one or more such epitopic regions or be sufficiently similar to such regions to retain their antigenic/immunogenic properties.

In an additional aspect of the invention there are provided antigenic/immunogenic fragments of the proteins or polypeptides of the invention, or of analogs or derivatives thereof.

Thus, what is important for analogs, derivatives and fragments is that they possess at least a degree of the antigenicity/immunogenicity of the protein or polypeptide from which they are derived.

Also included are polypeptides which have fused thereto other compounds which alter the polypeptides biological or pharmacological properties i.e. polyethylene glycol (PEG) to increase half-life; leader or secretory amino acid sequences for ease of purification; prepro- and pro- sequences; and (poly)saccharides.

Furthermore, in those situations where amino acid regions are found to be polymorphic, it may be desirable to vary one or more particular amino acids to more effectively mimic the different epitopes of the different Pseudomonas strains.

Moreover, the polypeptides of the present invention can be modified by terminal -NH₂ acylation (eg. by acetylation, or thioglycolic acid amidation, terminal carboxy amidation, e.g. with ammonia or methylamine) to provide stability, increased hydrophobicity for linking or binding to a support or other molecule.

Also contemplated are hetero and homo polypeptide multimers of the polypeptide fragments and analogs. These polymeric forms include, for example, one or more polypeptides that have been cross-linked with cross-linkers such as avidin/biotin, gluteraldehyde or dimethylsuperimidate. Such polymeric forms also include polypeptides containing two or more tandem or inverted contiguous sequences, produced from multicistronic mRNAs generated by recombinant DNA technology.

In a further embodiment, the present invention also relates to chimeric polypeptides which comprise one or more polypeptides or fragments or analogs thereof as defined in the figures of the present application.

In a further embodiment, the present invention also relates to chimeric polypeptides comprising two or more polypeptides having a sequence chosen from SEQ ID NOS: 2, 4, 6, 8, 10, 12 or fragments or analogs thereof; provided that the polypeptides are linked as to form a chimeric polypeptide.

In a further embodiment, the present invention also relates to chimeric polypeptides comprising two or more polypeptides comprising a sequence chosen from SEQ ID NOS: 2, 4, 6, 8, 10 or 12 provided that the polypeptides are linked as to form a chimeric polypeptide.

Preferably, a fragment, analog or derivative of a polypeptide of the invention will comprise at least one antigenic region i.e. at least one epitope.

In order to achieve the formation of antigenic polymers (i.e, synthetic multimers), polypeptides may be utilized having bishaloacetyl groups, nitroarylhalides, or the like, where the reagents being specific for thio groups. Therefore, the link between two mercapto groups of the different polypeptides may be a single bond or may be composed of a linking group of at least two, typically at least four, and not more than 16, but usually not more than about 14 carbon atoms.

In a particular embodiment, polypeptide fragments and analogs of the invention do not contain a methionine (Met) starting residue. Preferably, polypeptides will not incorporate a leader or secretory sequence (signal sequence). The signal portion of a polypeptide of the invention may be determined according to established molecular biological techniques. In general, the polypeptide of interest may be isolated from a Pseudomonas culture and subsequently sequenced to determine the initial residue of the mature protein and therefore the sequence of the mature polypeptide.

Such an immunogenic fragment may include, for example, the polypeptide of the invention lacking an N-terminal leader peptide, and/or a transmembrane domain and/or external loops and/or turns.

The present invention further provides a fragment of the polypeptide comprising substantially all of the extra cellular domain of a polypeptide which has at least 70% identity, preferably 80% identity, more preferably 95% identity, to a second polypeptide comprising Seq. ID No. 2, 4, 6, 8, 10, 12 or fragments or analogs thereof, over the entire length of said sequence.

It is understood that polypeptides can be produced and/or used without their start codon (methionine or valine) and/or without their leader peptide to favor production and purification of recombinant polypeptides. It is known that cloning genes without sequences encoding leader peptides will restrict the polypeptides to the cytoplasm of E. coli and will facilitate their recovery (Glick, B.R. and Pasternak, J.J. (1998) Manipulation of gene expression in prokaryotes. In "Molecular biotechnology: Principles and applications of recombinant DNA", 2nd edition, ASM Press, Washington DC, p.109-143).

According to another aspect of the invention, there are also provided (i) a composition of matter containing a polypeptide of the invention, together with a carrier, diluent or adjuvant; (ii) a pharmaceutical composition comprising a polypeptide of the invention and a carrier, diluent or adjuvant; (iii) a vaccine comprising a polypeptide of the invention and a carrier, diluent or adjuvant; (iv) a method for inducing an immune response against Pseudomonas, in a host, by administering to the host, an immunogenically effective amount of a polypeptide of the invention to elicit an immune response, e.g., a protective immune response to Pseudomonas; and particularly, (v) a method for preventing and/or treating a Pseudomonas infection, by administering a prophylactic or therapeutic amount of a polypeptide of the invention to a host in need.

According to another aspect of the invention, there are also provided (i) a composition of matter containing a polynucleotide of the invention, together with a carrier, diluent or adjuvant; (ii) a pharmaceutical composition comprising a polynucleotide of the invention and a pharmaceutically acceptable carrier, diluent or adjuvant; (iii) a method for inducing an immune response against Pseudomonas, in a host, by administering to the host, an immunogenically effective amount of a polynucleotide of the invention to elicit an immune response, e.g., a protective immune response to Pseudomonas; and particularly, (iv) a method for preventing and/or treating a Pseudomonas infection, by administering a prophylactic or therapeutic amount of a polynucleotide of the invention to a host in need.

According to another aspect of the invention, there are also provided (i) a composition of matter containing a polypeptide of the invention, together with a liposome, carrier, diluent or adjuvant; (ii) a pharmaceutical composition comprising a polypeptide the invention and a liposome, carrier, diluent or adjuvant; (iii) a vaccine comprising a polypeptide of the invention and a liposome, carrier, diluent or adjuvant; (iv) a method for inducing an immune response against P. aeruginosa, in a host, by administering to the host, an immunogenically effective amount of a pharmaceutical composition of the invention to elicit an immune response, e.g., a protective immune response to P. aeruginosa; and particularly, (v) a method for preventing and/or treating a P. aeruginosa infection, by administering a prophylactic or therapeutic amount of a pharmaceutical composition of the invention to a host in need.

According to another aspect of the invention, there are also provided (i) a composition of matter containing a polynucleotide of the invention, together with a liposome, carrier, diluent or adjuvant; (ii) a pharmaceutical composition comprising a polynucleotide of the invention and a liposome, carrier, diluent or adjuvant; (iii) a method for inducing an immune response against P. aeruginosa, in a host, by administering to the host, an immunogenically effective amount of a pharmaceutical composition of the invention to elicit an immune response, e.g., a protective immune response to P. aeruginosa; and particularly, (iv) a method for preventing and/or treating a P. aeruginosa infection, by administering a prophylactic or therapeutic amount of a pharmaceutical composition of the invention to a host in need.

In a further embodiment, the polypeptides of the invention are associated with the liposomes.

As used herein, "associated with" means that the polypeptides of the invention are at least partially embedded in the liposome membrane, and preferably are not covalently linked to the lipids. The polypeptides may also be bonded to a lipid fatty acid "tail" which itself is embedded in the membrane.

In a further embodiment, the pharmaceutical compositions comprising a liposome associated with polypeptides in accordance with the present invention are antigenic.

In a further embodiment, the pharmaceutical compositions comprising a liposome associated with polypeptides in accordance with the present invention are immunogenic.

In a further embodiment, the pharmaceutical compositions comprising a liposome associated with polypeptides in accordance with the present invention can elicit an immune response in a host.

In a further embodiment, the present invention also relates to pharmaceutical compositions comprising a liposome associated with polypeptides which are able to raise antibodies having binding specificity to the polypeptides of the present invention as defined above.

In an additional aspect of the invention there are provided pharmaceutical compositions comprising a liposome associated with immunogenic and/or antigenic fragments of the polypeptides of the invention, or of analogs thereof.

The present invention further provides pharmaceutical compositions comprising a liposome associated with fragments which comprise a B-cell or T-helper epitope.

The present invention further provides pharmaceutical compositions comprising a liposome associated with fragment that may be part of a larger polypeptide. It can be advantageous to include an additional amino acid sequence which contains secretory or leader sequences, or sequences which aid in purification such as multiple histidine residues, or an additional sequence which increases stability during recombinant production, or an additional polypeptide or lipid tail sequences which increase the immunogenic potential of the final polypeptide.

The skilled person will appreciate that pharmaceutical compositions comprising a liposome associated with analogs of the polypeptides of the invention will also find use in the context of the present invention, i.e. as antigenic/immunogenic material. Thus, for instance proteins or polypeptides which include one or more additions, deletions, substitutions or the like are encompassed by the present invention.

In a further embodiment, the present invention also relates to pharmaceutical compositions comprising a liposome associated with chimeric polypeptides which comprise one or more polypeptides or fragments or analogs thereof of the invention.

Liposomes are made of phospholipids and other polar amphiles, which form closed concentric bilayer membranes [summarized in Gregoriades, G., Immunology Today, 11, 3, 89 (1990); Lasic, D., American Scientist, 80, p. 20 (1992); Remington's on Pharmaceutical Sciences, 18th ed., 1990, Mack Publishing Co., Pennsylvania., p.1691]. The primary constituent of liposomes are lipids, which have a polar hydrophilic "head" attached to a long, nonpolar, hydrophobic "tail". The hydrophilic head typically consists of a phosphate group, while the hydrophobic tail is made of two long hydrocarbon chains. Since the lipid molecules have one part that is water-soluble and another part that is not, they tend to aggregate in ordered structures that sequester the hydrophobic tails from water molecules. In the process, liposomes can entrap water and solutes in their interior, or molecules with hydrophobic regions can also be incorporated directly into the liposomal membranes. Many phospholipids, alone or in combination, with other lipids will form liposomes. By convention, liposomes are categorized by size, and a 3-letter acronym is used to designate the type of liposome being discussed. Multilamellar vesicles are designated "MLV", large unilamellar vesicles "LUV", small unilamellar vesicles "SUV". These designations are sometimes followed by the chemical composition of the liposome. Nomenclature and a summary of known liposomes is described in Storm et al, 1998, PSIT, 1:19-31. Liposomes are efficient in helping membrane proteins refolding and are also efficient adjuvant boosting the humoral as well as the cellular immune response against an antigen.

The invention provides pharmaceutical compositions comprising liposomes constituted from phospholipids. These phospholipids can be synthetized or extracted from bacterial cells, soybean, eggs.

The invention provides a process for the incorporation of polypeptides of the invention into different liposome formulations.

Liposomes can be prepared with various synthetic phospholipids (List 1) or bacterial phospholipids and/or cholesterol, which can be combined at different ratios.

The invention provides a method for extracting lipids from bacterial cells in order to generate liposome formulations from bacterial origin. Complex lipid mixtures can be extracted from several bacterial species. These species could include but are not limited to : Neisseria spp, Haemophilus spp, Pseudomonas spp, Bacteriodes spp, Legionella spp, Vibrio spp, Brucella spp, Bordetella spp, Campylobacter spp, Klebsiella spp, Salmonella spp, Shigella spp, Proteus spp, and Yersinia spp. Other species can be found in Bergey's Manual of Determinative Bacteriology (1974) (Baltimore).

The liposomes of the.invention can be prepared from a variety of vesicle-forming lipids including phosphatidyl ethers and esters, such as phosphatidylethanloamine (PE), phosphatidylserine (PS), phosphatidylglycerol (PG) and phosphatidylcholine (PC) but also from glycerides, such as dioleoylglycerosuccinate; cerebrosides; gangliosides, sphyngomyelin; steroids, such as cholesterol; and other lipids, as well as excipients such as Vitamin E or Vitamin C palmitate.

The fluidity and stability of the liposomal membrane will depend on the transition temperature (temperature at which hydrocarbon regions change from a quasicrystalline to a more fluid state) of the phospholipids.

Modifications of membrane fluidity, number of lamellae, vesicle size, surface charge, lipid to antigen ratio and localization of the antigen within the liposome can modulate the ajduvanticity of liposomal preparations.

The preparation of liposomes can be made by a number of different techniques including ethanol injection; ether infusion; detergent removal; solvent evaporation; evaporation of organic solvents from chloroform in water emulsions; extrusion of multilamellar vesicles through a nucleopore polycarbonate membrane; freezing and thawing of phospholipid mixtures, as well *as sonication and homogenization.*

Lipids can be dissolved in a suitable organic solvent or mixture of organic solvents, such as a chloroform:methanol solution in a round bottom glass flask and dried using a rotatory evaporator to achieve an even film on the vessel.

Before immunization, the polypeptides of the invention can also be coupled or conjugated to carrier proteins such as tetanus toxin, diphtheria toxin, hepatitis B virus surface antigen, poliomyelitis virus VP1 antigen or any other viral or bacterial toxin or antigen or any suitable proteins to stimulate the development of a stronger immune response. This coupling or conjugation can be done chemically or genetically. A more detailed description of peptide-carrier conjugation is available in Van Regenmortel, M.H.V., Briand J.P., Muller S., Plaué S., «Synthetic Polypeptides as antigens» in Laboratory Techniques in Biochemistry and Molecular Biology, Viol.19 (ed.) Burdou, R.H. & Van Knippenberg P.H. (1988), Elsevier New York.

According to another aspect, there are provided pharmaceutical compositions comprising one or more Pseudomonas polypeptides of the invention in a mixture with a pharmaceutically acceptable adjuvant. Suitable adjuvants include (1) oil-in-water emulsion formulation such as MF59™. SAF™, Ribi™; (2) Freund's complete or incomplete adjuvant; (3) salts i.e. AlK(SO₄)₂, AlNa(SO₄)₂, AlNH₄(SO₄)₂, Al(OH)₃, AlPO₄, silica, kaolin; (4) saponin derivatives such as Stimulon™ or particles generated therefrom such as ISCOMs (immunostimulating complexes); (5) cytoimes such as interleukins, interferons, macrophage colony stimulating factor (M-CSF), tumor necrosis factor (TNF); (6) other substances such as carbon polynucleotides i.e. poly IC and poly AU, detoxified cholera toxin (CTB)and E.coli heat labile toxin for induction of mucosal immunity; (7) liposomes. A more detailed description of adjuvants is available in a review by M.Z.I Khan et al. in Pharmaceutical Research, vol. 11, No. 1 (1994) pp2-11, and also in another review by Gupta et al., in Vaccine, Vol. 13, No. 14, pp1263-1276 (1995) and in WO 99/24578. Preferred adjuvants include QuilA™, QS21™, Alhydrogel™ and Adjuphos™.

Pharmaceutical compositions of the invention may be administered parenterally by injection, rapid infusion, nasopharyngeal absorption, dermoabsorption, or buccal or oral.

The term "pharmaceutical composition" is also meant to include antibodies. In accordance with the present invention, there is also provided the use of one or more antibodies having binding specificity for the polypeptides of the present invention for the treatment or prophylaxis of Pseudomonas infection and/or diseases and symptoms mediated by Pseudomonas infection.

Pharmaceutical compositions of the invention are used for the prophylaxis of Pseudomonas infection and/or diseases and symptoms mediated by Pseudomonas infection as described in Manual of Clinical Microbiology, P.R. Murray (Ed, in chief), E.J. Baron, M.A. Pfaller, F.C. Tenover and R.H. Yolken. ASM Press, Washington, D.C. seventh edition, 1999, 1773p. and in Campa, M. et al. (Eds.) Pseudomonas aeruginosa as. an opportunistic pathogen (1993) Plenum Press, NY, 419 p.

In one embodiment, pharmaceutical compositions of the present invention are used for the treatment or nosocomial infections, especially in immunocompromised individuals such as ventilator-associated pneumonia in intubated patients, bacteremia in burned patients, chronical infection in cystic fibrosis patients and septicemia. In one embodiment, pharmaceutical compositions of the invention are used for the treatment or prophylaxis of Pseudomonas infection and/or diseases and symptoms mediated by Pseudomonas infection. In a further embodiment, the Pseudomonas infection is mediated by Pseudomonas aeruginosa. In a further embodiment, the Pseudomonas infection is mediated by Pseudomonas stutzeri.

In a particular embodiment, pharmaceutical compositions are administered to those hosts at risk of Pseudomonas infection such as infants, elderly and immunocompromised hosts and also hospitalized patients, cystic fibrosis patients, people susceptible to be burnt such as firemen, military personnel.

As used in the present application, the term "host" includes mammals. In a further embodiment, the mammal is human.

Pharmaceutical compositions are preferably in unit dosage form of about 0.001 to 100 µg/kg (antigen/body weight) and more preferably 0.01 to 10 µg/kg and most preferably 0.1 to 1 µg/kg 1 to 3 times with an interval of about 1 to 6 week intervals between immunizations.

Pharmaceutical compositions are preferably in unit dosage form of about 0.1 µg to 10 mg and more preferably 1µg to 1 mg and most preferably 10 to 100 µg 1 to 3 times with an interval of about 1 to 6 week intervals between immunizations.

According to another aspect, there are provided polynucleotides encoding polypeptides characterized by the amino acid sequence comprising a sequence chosen from SEQ ID NO: 4 or fragments or analogs thereof.

In one embodiment, polynucleotides are those illustrated in SEQ ID No: 3 which may include the open reading frames (ORF), encoding the polypeptides of the invention.

It will be appreciated that the polynucleotide sequences illustrated in the figures may be altered with degenerate codons yet still encode the polypeptides of the invention. Accordingly the present invention further provides polynucleotides which hybridize to the polynucleotide sequences herein above described (or the complement sequences thereof) having at least 90% identity between sequences. In a further embodiment, polynucleotides are hybridizable under stringent conditions i.e. having at least 95% identity. In a further embodiment, more than 97% identity. Suitable stringent conditions for hybridation can be readily determined by one of skilled in the art (see for example Sambrook et al., (1989) Molecular cloning : A Laboratory Manual, 2nd ed, Cold Spring Harbor, N.Y.; Current Protocols in Molecular Biology, (1999) Edited by Ausubel F.M. et al., John Wiley & Sons, Inc., N.Y.).

In a further embodiment, the present invention provides polynucleotides that hybridize under stringent conditions to either
(a) a DNA sequence encoding a polypeptide or
(b) the complement of a DNA sequence encoding a polypeptide;
wherein said polypeptide comprises a sequence chosen from SEQ ID NO: 4 or fragments or analogs thereof.

In a further embodiment, the present invention provides polynucleotides that hybridize under stringent conditions to either
(a) a DNA sequence encoding a polypeptide or
(b) the complement of a DNA sequence encoding a polypeptide;
wherein said polypeptide comprises at least 10 contiguous amino acid residues from a polypeptide comprising a sequence chosen from SEQ ID NO: 4 or fragments or analogs thereof.

In a further embodiment, the present invention provides polynucleotides that hybridize under stringent conditions to either
(a) a DNA sequence encoding a polypeptide or
(b) the complement of a DNA sequence encoding a polypeptide;
wherein said polypeptide comprises at least 20 contiguous amino acid residues from a polypeptide comprising a sequence chosen from SEQ ID NO: 4 or fragments or analogs thereof.

In a further embodiment, the present invention provides polynucleotides that hybridize under stringent conditions to either
(a) a DNA sequence encoding a polypeptide or
(b) the complement of a DNA sequence encoding a polypeptide;
wherein said polypeptide comprises at least contiguous amino acid residues from a polypeptide comprising a sequence chosen from SEQ ID NO: 4.

In a further embodiment, polynubleotides are those encoding polypeptides of the invention illustrated in SEQ ID NO: 4.

In a further embodiment, polynucleotides are those illustrated in SEQ ID NO: 3 encoding polypeptides of the invention.

As will be readily appreciated by one skilled in the art, polynucleotides include both DNA and RNA.

The present invention also includes polynucleotides complementary to the polynucleotides described in the present application.

According to another aspect, there is provided a process for producing polypeptides of the invention by recombinant techniques by expressing a polynucleotide encoding said polypeptide in a host cell and recovering the expressed polypeptide product. Alternatively, the polypeptides can be produced according to established synthetic chemical techniques i.e. solution phase or solid phase synthesis of oligopeptides which are ligated to produce the full polypeptide (block ligation).

General methods for obtention and evaluation of polynucleotides and polypeptides are described in the following references: Sambrook et al, Molecular Cloning: A Laboratory Manual, 2nd ed, Cold Spring Harbor, N.Y., 1989; Current Protocols in Molecular Biology, Edited by Ausubel F.M. et al., John Wiley and Sons, Inc. New York: PCR Cloning Protocols, from Molecular Cloning to Genetic Engineering, Edited by White B.A., Humana Press, Totowa, New Jersey, 1997, 490 page; Protein Purification, Principles and Practices, Scopes R.K., Springer-Verlag, New York, 3rd Edition, 1993, 380 pages; Current Protocols in Immunology, Edited by Coligan J.E. et al., John Wiley & Sons Inc., New York.

The present invention provides host cells transfected with vectors comprising the polynucleotides of the invention.

The present invention provides a process for producing a polypeptide comprising culturing a host cell of the invention under conditions suitable for expression of said polypeptide.

For recombinant production, host cells are transfected with vectors which encode the polypeptides of the invention, and then cultured in a nutrient media modified as appropriate for activating promoters, selecting transformants or amplifying the genes. Suitable vectors are those that are viable and replicable in the chosen host and include chromosomal, non-chromosomal and synthetic DNA sequences e.g. bacterial plasmids, phage DNA, baculovirus, yeast plasmids, vectors derived from combinations of plasmids and phage DNA. The polypeptide sequence may be incorporated in the vector at the appropriate site using restriction enzymes such that it is operably linked to an expression control region comprising a promoter, ribosome binding site (consensus region or Shine-Dalgarno sequence), and optionally an operator (control element). One can select individual components of the expression control region that are appropriate for a given host and vector according to established molecular biology principles (Sambrook et al, Molecular Cloning: A Laboratory Manual, 2nd ed, Cold Spring Harbor, N.Y., 1989; Current Protocols in Molecular Biology, Edited by Ausubel F.M. et al., John Wiley and Sons, Inc. New York). Suitable promoters include but are not limited to LTR or SV40 promoter, E. coli lac, tad or trp promoters and the phage lambda P_{L} promoter. Vectors will preferably incorporate an origin of replication as well as selection markers i.e., ampicillin resistance gene. Suitable bacterial vectors include pET, pQE70, pQE60, pQE-9, pD10 phagescript, psix174, pbluescript Sk, pbsks, pNH8A, pNH16a, pNH18A, pNH46A, ptrc99a, pKK223-3, pKK233-3, pDR540, pRIT5 and eukaryotic vectors pBlueBacIII, pWLNEO, pSV2CAT, pOG44, pXT1, pSG, pSVK3, pBPV, pMSG and pSVL. Host cells may be bacterial i.e. E. coli, Bacillus subtilis, Streptomyces; fungal i.e. Aspergillus niger, Aspergillus nidulins; yeast i.e. Saccharomyces or eukaryotic i.e. CHO, COS.

Upon expression of the polypeptide in culture, cells are typically harvested by centrifugation then disrupted by physical or chemical means (if the expressed polypeptide is not secreted into the media) and the resulting crude extract retained to isolate the polypeptide of interest. Purification of the polypeptide from culture media or lysate may be achieved by established techniques depending on the properties of the polypeptide i.e. using ammonium sulfate or ethanol precipitation, acid extraction, anion or cation exchange chromatography, phosphocellulose chromatography, hydrophobic interaction chromatography, hydroxylapatite chromatography and lectin chromatography. Final purification may be achieved using HPLC.

The polypeptides may be expressed with or without a leader or secretion sequence. In the former case the leader may be removed using post-translational processing (see US 4,431,739; US 4,425,437; and US 4,338,397) or be chemically removed subsequent to purifying the expressed polypeptide.

According to a further aspect, the Pseudomonas polypeptides of the invention may be used in a diagnostic test for Pseudomonas infection, in particular Pseudomonas aeruginosa infection.

Several diagnostic methods for Pseudomonas infection in an host susceptible to Pseudomonas infection are possible, for example detecting Pseudomonas organism in a biologidal sample, the following procedure may be followed:
a) obtaining a biological sample from a host;
b) incubating an antibody or fragment thereof reactive with a Pseudomonas polypeptide of the invention with the biological sample to form a mixture; and
c) detecting specifically bound antibody or bound fragment in the mixture which indicates the presence of Pseudomonas.

Alternatively, a method for diagnostic for Pseudomonas infection in an host susceptible to Pseudomonas infection includes a method for the detection of antibody specific to a Pseudomonas antigen in a biological sample containing or suspected of containing said antibody may be performed was follows:
a) obtaining a biological sample from a host;
b) incubating one or more Pseudomonas polypeptides of the invention or fragments thereof with the biological sample to form a mixture; and
c) detecting specifically bound antigen or bound fragment in the mixture which indicates the presence of antibody specific to Pseudomonas.

One of skill in the art will recognize that this diagnostic test may take several forms, including an immunological test such as an enzyme-linked immunosorbent assay (ELISA), a radioimmunoassay or a latex agglutination assay, essentially to determine whether antibodies specific for the polypeptide are present in an organism.

The DNA sequences encoding polypeptides of the invention may also be used to design DNA probes for use in detecting the presence of Pseudomonas in a biological sample suspected of containing such bacteria. The detection method of this invention comprises:
a) obtaining the biological sample from a host;
b) incubating one or more DNA probes having a DNA sequence encoding a polypeptide of the invention or fragments thereof with the biological sample to form a mixture; and
c) detecting specifically bound DNA probe in the mixture which indicates the presence of Pseudomonas bacteria.

The DNA probes of this invention may also be used for detecting circulating Pseudomonas i.e. Pseudomonas nucleic acids in a sample, for example using a polymerase chain reaction; as a method of diagnosing Pseudomonas infections. The probe may be synthesized using conventional techniques and may be immobilized on a solid phase, or may be labelled with a detectable label. A preferred DNA probe for this application is an oligomer having a sequence complementary to at least about 6 contiguous nucleotides of the Pseudomonas polypeptides of the invention. In a further embodiment, the preferred DNA probe will be an oligomer having a sequence complementary to at least about 15 contiguous nucleotides of the Pseudomonas polypeptides of the invention. In a further embodiment, the preferred DNA probe will be an oligomer having a sequence complementary to at least about 30 contiguous nucleotides of the Pseudomonas polypeptides of the invention. In a further embodiment, the preferred DNA probe will be an oligomer having a sequence complementary to at leat about 50 contiguous nucleotides of the Pseudomonas polypeptides of the invention.

Another diagnostic method for the detection of Pseudomonas in a host comprises :
a) labelling an antibody reactive with a polypeptide of the invention or fragment thereof with a detectable label;
b) administering the labelled antibody or labelled fragment to the host; and
c) detecting specifically bound labelled antibody or labelled fragment in the host which indicates the presence of Pseudomonas.

In a further aspect, polynucleotides encoding polypeptides of the invention, or fragments, analogs or derivatives thereof, may be used in a DNA immunization method. That is, they can be incorporated into a vector which is replicable and expressible upon injection thereby producing the antigenic polypeptide in vivo. For example polynucleotides may be incorporated into a plasmid vector under the control of the CMV promoter which is functional in eukaryotic cells. Preferably the vector is injected intramuscularly.

A further aspect of the invention is the use of the Pseudomonas polypeptides of the invention as immunogens for the production of specific antibodies for the diagnosis and in particular the treatment of Pseudomonas infection.

A further aspect of the invention is the use of the antibodies directed to the polypeptides of the invention for passive immunization, whereby an antibody raised by a polypeptide of the invention is administered to a host in an amount sufficient to provide a passive immunization. One could use the antibodies described in the present application. Suitable antibodies may be determined using appropriate screening methods, for example by measuring the ability of a particular antibody to passively protect against Pseudomonas infection in a test model. One example of an animal model is the mouse model described in the examples herein. The antibody may be a whole antibody or an antigen-binding fragment thereof and may belong to any immunoglobulin class. The antibody or fragment may be of animal origin, specifically of mammalian origin and more specifically of murine, rat or human origin. It may be a natural antibody or a fragment thereof, or if desired, a recombinant antibody or antibody fragment. The term recombinant antibody or antibody fragment means antibody or antibody fragment which was produced using molecular biology techniques. The antibody or antibody fragments may be polyclonal, or preferably monoclonal. It may be specific for a number of epitopes associated with the Pseudomonas polypeptides but is preferably specific for one.

The use of a polynucleotide of the invention in. genetic immunization will preferably employ a suitable delivery method or system such as direct injection of plasmid DNA into muscles [Wolf et al. H M G (1992) 1: 363; Turnes et al., Vaccine (1999), 17 : 2089; Le et al., Vaccine (2000) 18 : 1893; Alves et al., Vaccine (2001) 19 : 788], injection of plasmid DNA with or without adjuvants [Ulmer et al., Vaccine (1999) 18: 18; MacLaughlin et al., J. Control Release (1998) 56: 259; Hartikka et al., Gene Ther. (2000) 7: 1171-82; Benvenisty and Reshef, PNAS USA (1986) 83:9551; Singh et al., PNAS USA (2000) 97: 811], targeting cells by delivery of DNA complexed with specific carriers [Wa et al., J Biol Chem (1989) 264: 16985; Chaplin et al., Infect. Immun. (1999) 67: 6434], injection of plasmid complexed or encapsulated in various forms of liposomes [Ishii et al., AIDS Research and Human Retroviruses (1997) 13: 142; Perrie et al., Vaccine (2001) 19: 3301], administration of DNA with different methods of bombardment [Tang et al., Nature (1992) 356: 152; Eisenbraun et al., DNA Cell Biol (1993) 12: 791; Chen et al., Vaccine (2001) 19: 2908], and administration of DNA with lived vectors [Tubulekas et al., Gene (1997) 190: 191; Pushko et al., Virology (1997) 239: 389; Sprerig et al. FEMS (2000) 27: 299; Dietrich et al., Vaccine (2001) 19: 2506].

In a further aspect, the invention provides a method for prophylactic or therapeutic treatment of Pseudomonas infection in a host susceptible to Pseudomonas infection comprising administering to the host a prophylactic or therapeutic amount of a pharmaceutical composition of the invention.

In a further embodiment, the invention provides the use of a pharmaceutical composition of the invention in the manufacture of a medicament for the prophylactic or therapeutic treatment of Pseudomonas infection.

In a further embodiment, the invention provides a kit comprising a polypeptide of the invention for detection or diagnosis of Pseudomonas infection.

Unless otherwise defined, all technical aad scientific terms used herein have the same meaning as commonly understood by one of ordinary skill in the art to which this invention belongs. In addition, the materials, methods, and examples are illustrative only and not intended to be limiting.

### EXAMPLE 1

This example illustrates the cloning and molecular characteristics of SPA-1 gene and corresponding polypeptide.

The coding region of P. aeruginosa SPA-1 (SEQ ID NO: 1) gene was amplified by PCR (Hybaid PCR Express, ESBE Scientific, Markham, Ontario, Canada) from genomic DNA of P. aeruginosa strain PAO1 using the following oligos that contained base extensions for the addition of restriction sites *Nde*I (CATATG) and *Not*I (GCGGCCGC): PSEU59 (5' - GGGAATTCCATATGGCGCAGAAGAATCCGACAGTCG -3') and PSEU60 (5' - ATAAGAATGCGGCCGCTGGCGTCCQCAGGCGGT -3'). PCR products were purified from agarose gel using a QIAquick gel extraction kit following the manufacturer's instructions (Qiagen, Chatsworth, CA), and digested with *Nde*I and *Not*I (Amersham Pharmacia Biotech, Inc, Baie d'Urfé, Canada). The pET21b(+) vector (Novagen, Madison, WI) was digested with *Nde*I and *Not*I and purified from agarose gel using a QIAquick gel extraction kit (Qiagen). The *Nde*I-*Not*I PCR products were ligated to the *Nde*I-*Not*I pET21b(+) expression vector. The ligated products were transformed into E. coli strain DH5α [φ80d*lac*ZΔM15 Δ(*lac*ZYA-*arg*F) U169 *end*A1 *rec*A1 *hsd*R17 (rₖ-mₖ+) *deo*R *thi*-1 *sup*E44 λ⁻*gyr*A96 *rel*A1] (Gibco BRL, Gaithersburg, MD) according to the method of Simanis (Hanahan, D. DNA Cloning, 1985, D.M. Glover (ed), pp. 109-135). Recombinant pET21b(+) plasmid (rpET21b(+)) containing *SPA-1 gene was purified using a Qiagen kit and DNA insert* was sequenced (Taq Dye Deoxy Terminator Cycle Sequencing kit, ABI, Foster City, CA).

**Table 1. Oligonucleotide primers used for PCR amplification of P. aeruginosa genes.**

| **Genes** | **Primers I.D.** | **Restriction site** | **Vector** | **Sequence** | **Sequence I.D. No.** |
|---|---|---|---|---|---|
| SPA-1 | PSEU59 | *Nde*I | pET21b (+) | | 7 |
| SEPA-1 | PSEU60 | *Not*I | pET21b (+) | | 8 |
| SPA-1 | PSEU409 | *Bgl*II | PCMV-GH | | 9 |
| SPA-1 | PSEU410 | *Xba*I | pCMV-GH | | 10 |
| SPA-2 | PSEU47 | *Nde*I | pET21b (+) | | 11 |
| SPA-2 | PSEU48 | *Hin*dIII | pET21b (+) | | 12 |
| SPA-2 | PSEU411 | *Bam*HI | pCMV-GH | | 13 |
| SPA-2 | PSEU412 | *Hin*dIII | pCMV-GH | | 14 |
| SPA-3 | PSEU37 | *Nde*I | pET21b (+) | | 15 |
| SPA-3 | PSEU38 | HindIII | pET21b (+) | | 16 |
| SPA-3 | PSEU413. | BamHI | pCMV-GH | | 17 |
| SPA-3 | PSEU414 | HindIII | pCMV-GH | | 18 |

It was determined that the open reading frame (ORF) which codes for SPA-1 polypeptide contains 1347 bp and encodes a 448 amino acid residues polypeptide with a predicted pI of 8.20 and a predicted molecular mass of 47757.95 Da. Analysis of the predicted amino acid residues sequence (SEQ ID NO :2) using the Spscan software (Wisconsin Sequence Analysis Package; Genetics Computer Group) suggested the existence of a 32 amino acid residues signal peptide (MRNPERSALLKVSGLLGSTVVAMGLGLSSACA), which ends with a cleavage site located between an alanine and a glutamine residues.

To confirm the presence by PCR amplification of SPA-1 (SEQ ID NO:1) gene, the following 5 distinct P. aeruginosa strains were used: P. aeruginosa PAO1, NF25, NF45, 1019-5 and B. Clinical isolates were provided by the Centre de Recherche en Infectiologie (Laval University, Québec, Canada). The E. coli XL1-Blue MRF' was used in these experiments as a negative control. SPA-1 (SEQ ID NO :1) gene was amplified by PCR (Hybaid PCR Express, ESBE Scientific) from genomic DNA from the 5 P. aeruginosa strains, and the control E. coli strain using the oligonucleotides primers PSEU59 and PSEU60 (Table 1). PCR was performed with 10 cycles of 10 sec at 94°C, 30 sec at 45°C and 2 min at 68°C followed by 20 cycles of 10 sec at 94°C, 30 sec at 45°C and 2 min with 0.05 sec increments per cycle at 68°C and a final elongation period of 7 min at 68°C. The PCR products were size fractionated in 1% agarose gels and were visualized by ethidium bromide staining. The results of these PCR amplifications are presented in Table 2. The analysis of the amplification products revealed that SPA-1 (SEQ ID NO :1) gene was present in the genome of all of the 5 P. aeruginosa strains tested. No such product was detected when the control E. coli DNA was submitted to identical *PCR amplifications* with these oligonucleotide primers.

**Table 2. Identification of P. aeruginosa genes by PCR amplification.**

| **Strain Identification** | **Identification by PCR amplification of** | | |
|---|---|---|---|
| | SPA-1 | SPA-2 | SPA-3 |
| PAO1 | + | + | + |
| NF25 | + | + | + |
| NF45 | + | + | + |
| 1019-5 | + | + | + |
| B | + | + | + |
| E. coli | - | - | - |

### EXAMPLE 2

This example illustrates the cloning and molecular characteristics of SPA-2 gene and corresponding polypeptide.

The coding region of P. aeruginosa SPA-2 (SEQ ID NO: 3) gene was amplified by PCR (Hybaid PCR Express, ESBE Scientific) from genomic DNA of P. aeruginosa strain PAO1 using the following oligos that contained base extensions for the addition of restriction sites *Nde*I (CATATG) and *Hind*III (AAGCTT): PSEU47 and PSEU48, which are presented in Table 1. The methods used for cloning SPA-2 gene into an expression vector and sequencing are similar to the methods described in Example 1.

It was determined that the open reading frame (ORF) which codes for SPA-2 contains 624 bp and encodes a 207 amino acid residues polypeptide with a predicted pI of 5.04 and a predicted molecular mass of 22882.24 Da. Analysis of the predicted amino acid residues sequence (SEQ ID NO :4) using the Spscan software (Wisconsin Sequence Analysis Package; Genetics Computer Group) suggested the existence of a 19 amino acid residues signal peptide (MKRILTSAALIGMTTLLAA), which ends with a cleavage site located between an alanine and a cysteine residues.

The SPA-2 gene was shown to be present after PCR amplification using the oligonucleotide primers PSEU47 and PSEU48 in the 5 P. aeruginosa strains tested (Table 2). The methods used for PCR amplification of the SPA-2 gene were similar to the methods presented in Example 1. No such product was detected when the control E. coli DNA was submitted to identical PCR amplification with these oligonucleotide primers.

### EXAMPLE 3

This example illustrates the cloning and molecular characteristics of SPA-3 gene and corresponding polypeptide.

The coding region of P. aeruginosa SPA-3 (SEQ ID NO: 5) gene was amplified by PCR (Hybaid PCR Express, ESBE Scientific) from genomic DNA of P. aeruginosa strain PAO1 using the following oligos that contained base extensions for the addition of restriction sites *Nde*I (CATATG) and *Hind*III (AAGCTT): PSEU37 and PSEU38, which are presented in Table 1. The methods used for cloning SPA-3 gene into an expression vector and sequencing are similar to the methods described in Example 1.

It was determined that the open reading frame (ORF) which codes for SPA-3 contains 1143 bp and encodes a 380 amino acid residues polypeptide with a predicted pI of 5.15 and a predicted molecular mass of 40394.19 Da. Analysis of the predicted amino acid residues sequence (SEQ ID NO :6) using the Spscan software (Wisconsin Sequence Analysis Package; Genetics Computer Group) suggested the existence of a 21 amino acid residues signal peptide (MVQWKHAALLALALAWGCSS), which ends with a cleavage site located between a serine and an asparagine residues.

The SPA-3 gene was shown to be present after PCR amplification using the oligonucleotide primers PSEU37 and PSEU38 in the 5 P. aeruginosa strains tested (Table 2). The methods used for PCR amplification of the SPA-3 gene were similar to the methods presented in Example 1. No such product was detected when the control E. coli DNA was submitted to identical PCR amplification with these oligonucleotide primers.

### EXAMPLE 4.

This example illustrates the cloning of P. aeruginosa genes in CMV plasmid pCMV-GH.

The DNA coding regions of P. aeruginosa polypeptides were inserted in phase downstream of a human growth hormone (hGH) gene which was under the transcriptional control of the cytomegalovirus (CMV) promotor in the plasmid vector pCMV-GH (Tang et al., Nature, 1992, 356 :152). The CMV promoter is non-functional in E. coli cells but active upon administration of the plasmid in eukaryotic cells. The vector also incorporated the ampicillin resistance gene.

The coding regions of SPA-1 (SEQ ID NO: 1), SPA-2 (SEQ ID NO: 3) and SPA-3 (SEQ ID NO: 5) genes without their leader peptide regions were amplified by PCR (Hybaid PCR Express, ESBE Scientific) from genomic DNA of P. aeruginosa strain PAO1 using oligonucleotide primers that contained base extensions for the addition of restriction sites *Bam*HI (GGATCC), *Bgl*II (AGATCT), *Xba*I (TCTAGA), or *Hind*III (AAGCTT) which are described in Table 1. The PCR products were purified from agarose gel using a QIAquick gel extraction kit (Qiagen), and digested with restriction enzymes (Amersham Pharmacia Biotech, Inc). The pCMV-GH vector (Laboratory of Dr. Stephen A. Johnston, Department of Biochemistry, The University of Texas, Dallas, Texas) was digested with *Bam*HI, *Bgl*II, XbaI, or *Hind*III and purified from agarose gel using the QIAquick gel extraction kit (Qiagen). The digested DNA fragments were ligated to the digested pCMV-GH vector to create the hGH-SPA-1, hGH-SPA-2 and hGH-SPA-3 fusion polypeptides under the control of the CMV promoter. The ligated products were transformed into E. coli strain DH5α [φ80d*lac*ZΔM15 Δ(*lac*ZYA-*arg*F) U169 *end*A1 *rec*A1 *hsd*R17 (rₖ-mₖ+) *deo*R *thi*-1 *sup*E44 λ⁻*gyr*A96 *rel*A1] (Gibco BRL) according to the method of Simanis (Hanahan, D. DNA Cloning, 1985, D.M. Glover (ed), pp. 109-135). The recombinant pCMV plasmids were purified using a Qiagen kit, and the nucleotide sequences of the DNA inserts were verified by DNA sequencing.

### EXAMPLE 5

This example illustrates the use of DNA to elicit an immune response to P. aeruginosa polypeptide antigens.

A group of 8 female BALB/c mice (Charles River, St-Constant, Québec, Canada) were immunized by intramuscular injection of 100 µl three times at two- or three-week intervals with 50 µg of recombinant pCMV-GH encoding SPA-1 (SEQ ID NO: 1), SPA-2 (SEQ ID NO: 3) and SPA-3 (SEQ ID NO: 5) genes in presence of 50 µg of granulocyte-macrophage colony-stimulating factor (GM-CSF)-expressing plasmid pCMV-GH-GM-CSF (Laboratory of Dr. Stephen A. Johnston, Department of Biochemistry, The University of Texas, Dallas, Texas). As control, a group of mice were injected with 50 µg of pCMV-GH in presence of 50 µg of pCMV-GH-GM-CSF. Blood samples were collected from the orbital sinus prior to each immunization and seven days following the third injection. Serum antibody responses were determined by ELISA using the corresponding His-Tag labeled P. aeruginosa recombinant polypeptides as coating antigen. The production and purification of these His-tag labeled P. aeruginosa recombinant polypeptides are presented in Example 6.

### EXAMPLE 6

This example illustrates the production and purification of P. aeruginosa recombinant polypeptides.

The recombinant pET21b(+) plasmid with SPA-1 (SEQ ID NO: 1), SPA-2 (SEQ ID NO: 3) and SPA-3 (SEQ ID NO: 5) genes were used to transform by electroporation (Gene Pulser II apparatus, BIO-RAD Labs, Mississauga, Canada) E. coli strain Tuner (DE3) [F⁻ *ampT hsdS_{B}* (r_{B}⁻ m_{B}⁻) *gal dcm lacY1* (DE3)] (Novagen). In this strain of E. coli, the T7 promotor controlling expression of the recombinant polypeptide is specifically recognized by the T7 RNA polymerase (present on the λDE3 prophage) whose gene is under the control of the lac promoter which is inducible by isopropyl-ß-d-thio-galactopyranoside (IPTG). The transformant Tuner (DE3)/ rpET21 was grown at 37°C with agitation at 250 rpm in Luria-Betani (LB) broth (peptone 10g/L, yeast extract 5g/L, NaCl 10g/L) containing 100 µg of ampicillin (Sigma-Aldrich Canada Ltd., Oakville, Canada) per ml until the A₆₀₀ reached a value of 0.5. In order to induce the production of His-tagged P. aerugionsa recombinant polypeptides, the cells were incubated for 3 additional hours in the presence of IPTG at as final concentration of 1 mM. Induced cells from a 1-L culture were pelleted by centrifugation and frozen at -70°C.

The purification of the recombinant polypeptides from the soluble or insoluble cytoplasmic fractions of IPTG-induced Tuner (DE3) /rpET21 was done by affinity chromatography based on the properties of the His•Tag sequence (6 consecutive histidine resides) to bind to divalent cations (Ni²⁺) immobilized on the His•Bind mental chelation resin. Briefly, for the purification of SPA-2 and SPA-3 polypeptides from the soluble cytoplasmic fraction, the pelleted cells obtained from a 1-L culture induced with IPTG were sonicated and centrifuged at 21,000 X g for 30 min to remove debris.. For the purification of recombinant polypeptides SPA-1 from the insoluble cytoplasmic fraction, the cells were sonicated and centrifuged as above and the resulting pellet was resuspended in lysis buffer (5 mM imidazole, 2 M NaCl, 20 mM Tris-HCl pH 7.9) with 6 M Guanidine-HCl. The suspension was incubated on ice for 1 h and centrifuged at 39,000 x g for 20 min. The supernatants containing soluble SPA-2 and SPA-3 polypeptides or solubilized SPA-1 polypeptide were deposited on a Ni-NTA agarose column (Qiagen). The His-tag labeled P. aeruginosa recombinant polypeptides were eluted with 250 mM imidazole-500mM NaCl-20 mM Tris pH 7.9. The removal of the salt and imidazole from the sample was done by dialysis against PBS at 4°C. The quantities of recombinant polypeptides obtained from the soluble or insoluble fractions of E. coli was estimated by MicroBCA (Pierce, Rockford, Illinois).

### EXAMPLE 7

This example illustrates the reactivity of the His-tagged P. aeruginosa recombinant polypeptides with antibodies present in human sera.

As shown in Table 3, SPA-1, SPA-2 and SPA-3 His-tagged recombinant polypeptides where recognized in immunoblots by the antibodies present in the human sera. It indicates that humans, which are normally in contact with P. aeruginosa, do develop antibodies that are specific to these polypeptides. These particular humans antibodies might be implicated in the protection against P. aeruginosa infection.

**Table 3. Reactivity in immunoblots of antibodies present in human sera with P. aeruginosa His-tagged fusion recombinant polypeptides.**

| **Purified recombinant polypeptide I.D.¹** | **Apparent molecular weight (kDa)²** | **Reactivity in immunoblots with antibodies present in human sera³** |
|---|---|---|
| SPA-1 | 48 | + |
| SPA-2 | 25 | + |
| SPA-3 | 40 | + |

| | | |
|---|---|---|
| ¹His-tagged recombinant polypeptides produced and purified as described in Example 6 were used to perform the immunoblots. ²Molecular weight of the His-tagged recombinant polypeptide was estimated after SDS-PAGE. ³A pool of three human *sera*, each diluted 1/500, was prepared in order to perform the immunoblots. | | |

### EXAMPLE 8

This example illustrates the accessibility to antibodies of the SPA-1, SPA-2 and SPA-3 polypeptides at the surface of P. aeruginosa strain.

Bacteria were grown overnight on blood agar at 30°C. Colonies were resuspended in LB broth to obtain, an O.D.₆₀₀ₙₘ of 0.3. Dilutions of anti-SPA-1, anti-SPA-2 or anti-SPA-3 or control sera were then added and allowed to bind to the cells, which were incubated four 2 h at 4°C with rotation. Samples were washed 2 times in blocking buffer (phosphate-buffered saline (PBS) containing 2% bovine serum albumin (BSA)], and then 500 µl of goat fluorescein (FITC)-conjugated anti-mouse IgG Fc (gamma) fragment-specific, diluted in blocking buffer, was added. After an additional incubation of 2 h at 4°C with rotation in the dark, samples were washed 2 times in blocking buffer and fixed with 0.25 % formaldehyde in PBS buffer for 18 h at 4°C. Cells were centrifuged and resuspended in 0.5 ml of PBS buffer. Cells were kept in the dark at 4°C until analyzed by flow cytometry (Epics® XL; Beckman Coulter, Inc.). Flow cytometric analysis revealed that SPA-1-, SPA-2-, and SPA-3-specific antibodies efficiently recognized their corresponding surface-exposed epitopes on the homologous (PAO1) P. aeruginosa strain tested (Table 4). It was determined that more than 55 % of the 10,000 Pseudomonas cells analyzed were labeled with the antibodies present in the SPA-1-, SPA-2-, and SPA-3-specific sera. These observations clearly demonstrate that the SPA-1, SPA-2 and SPA-3 polypeptides are accessible at the surface, where they can be easily recognized by antibodies. Anti-P. aeruginosa antibodies were shown to play an important role in the protection against P. aeruginosa infection.

**Table 4. Evaluation of the attachment of SPA-1-, SPA-2- and SPA-3-specific antibodies at the surface of intact cells of P. aeruginosa strain PA01.**

| **Serum Identification** | **Fluorescence Index²** | **% of labeled cells³** |
|---|---|---|
| SPA-1-specific sera¹ | 10.0 | 58 |
| SPA-2-specific sera | 21.3 | 75 |
| SPA-3-specific sera | 10.0 | 55 |
| Negative control sera⁴ | 1.0 | 1.0 |
| Positive control serum⁵ | 37.4 | 83 |

| | | |
|---|---|---|
| ¹ Mice were injected subcutaneously four times at two-week intervals with 20 µg of purified recombinant polypeptides mixed with 10 µg of QuilA adjuvant (Cedarlane Laboratories, Hornby, Canada). Sera were diluted 1/50. ² The fluorescence index was calculated as the median fluorescence value obtained after labelling the cells with an immune serum divided by the fluorescence value obtained for a control mouse serum. A fluorescence value of 1 indicated that there was no binding of antibodies at the surface of intact Pseudomonas cells. ³ % of labeled cells out of the 10,000 cells analyzed. ⁴ Sera collected from unimmunized or sham-immunized mice were pooled, diluted 1/50, and used as negative control for this assay. ⁵ Serum obtained from a mouse immunized with 20 µg of purified recombinant outer membrane polypeptide OprI from P. aeruginosa strain PAO1 was diluted 1/50 and used as a positive control for the assay. | | |

### EXAMPLE 9

This example illustrates the protection of mice against P. aeruginosa infection induced by immunization with SPA-2 recombinant polypeptide.

Groups of 4 female BALB/c mice (Charles River) were immunized subcutaneously four times at two-week intervals with 20 µg of affinity purified His-tagged P. aeruginosa SPA-2 recombinant polypeptide in presence of 10% of QuilA adjuvant (Cedarlane Laboratories Ltd) or, as control, with QuilA adjuvant alone in PBS. Blood samples were collected from the orbital sinus on day 0, 14, 28, and 42 prior to each immunization and 7 days (day 49) following the fourth injection. One week later, the mice were challenged intratrachealy with approximately 5×10⁷ CFU of P. aeruginosa strain PAO1. Samples of the P. aeruginosa challenge inoculum were plated on blood agar plates to determine the CPU and to verify the challenge dose. Mice survival was monitored on a 5-day period and protection was reported as the percentage of surviving mice compared to survival in the group of mice immunized with adjuvant only. Results reported in Table 5 indicate that immunization with SPA-2 recombinant polypeptide can delay mortality and protect mice from a lethal Pseudomonas infection.

**Table 5. Protection confered by immunization with SPA-2 recombinant polypeptide against an intratracheal lethal challenge.**

| **Groups¹** | **% Survival** | **Mean Survival Time** |
|---|---|---|
| SPA-2 | 75 | 108 h |
| QuilA | 25 | 75 h |

| | | |
|---|---|---|
| ¹ Mice were injected subcutaneously four times at two-week intervals with 20 µg of purified recombinant polypeptide mixed with 10 µg of QuilA adjuvant (Cedarlane Laboratories, Hornby, Canada); or with QuilA adjuvant only as a negative control. | | |

### Example 10. This example illustrates the identification of SPA-1 homologs, in the Pseudomonas aeruginosa genome, which can be used as immunogens for vaccines.

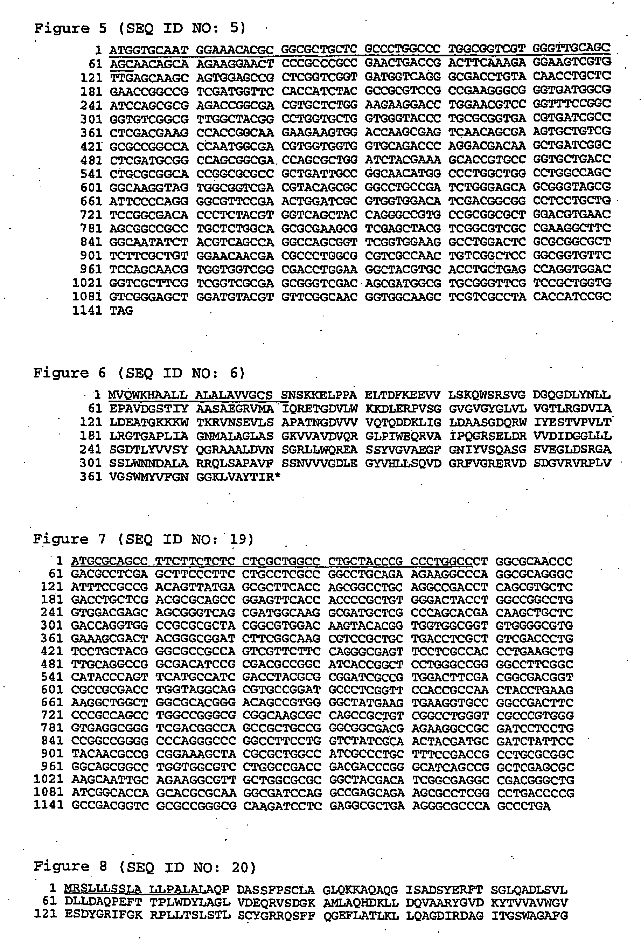

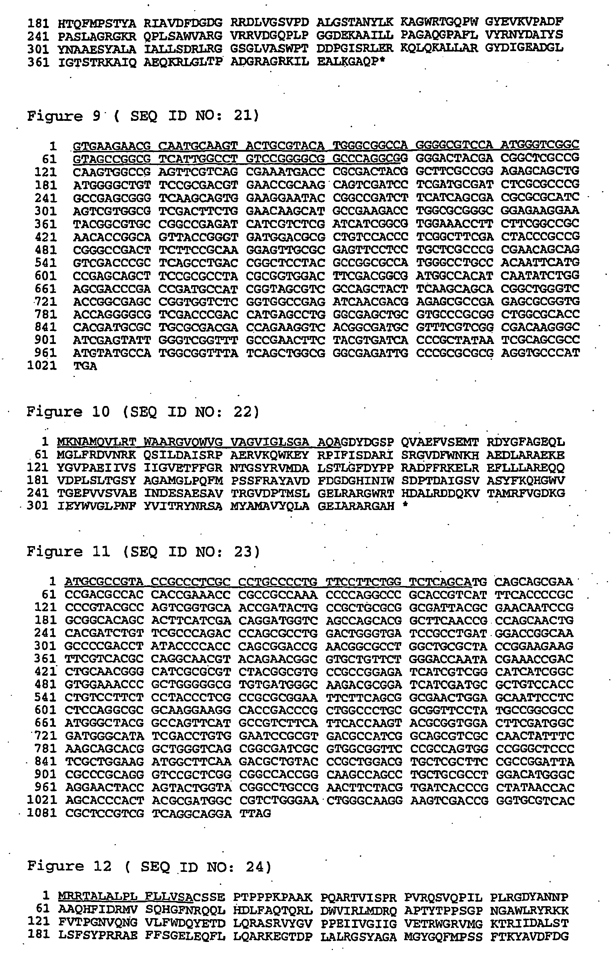

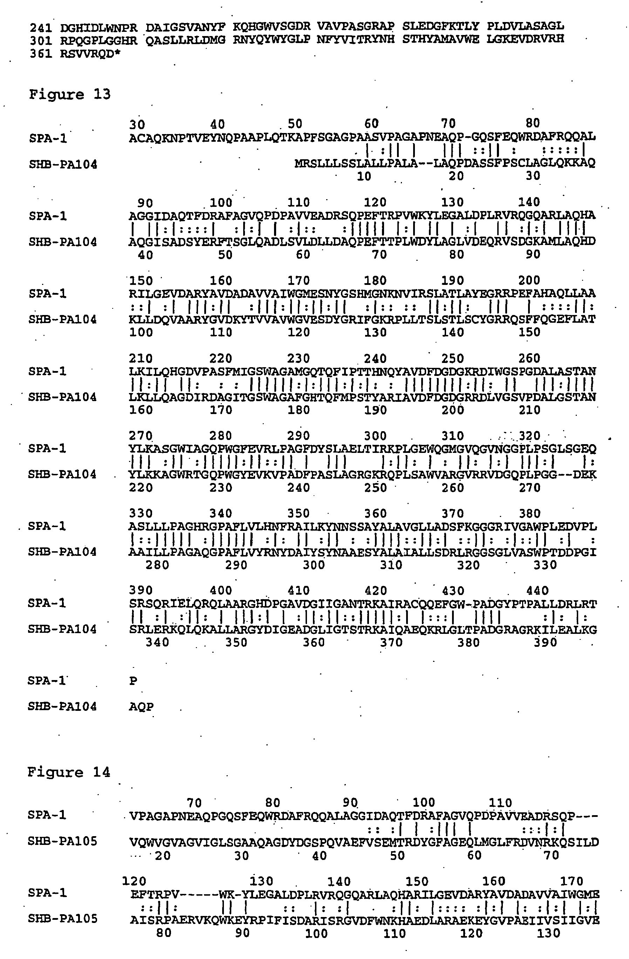

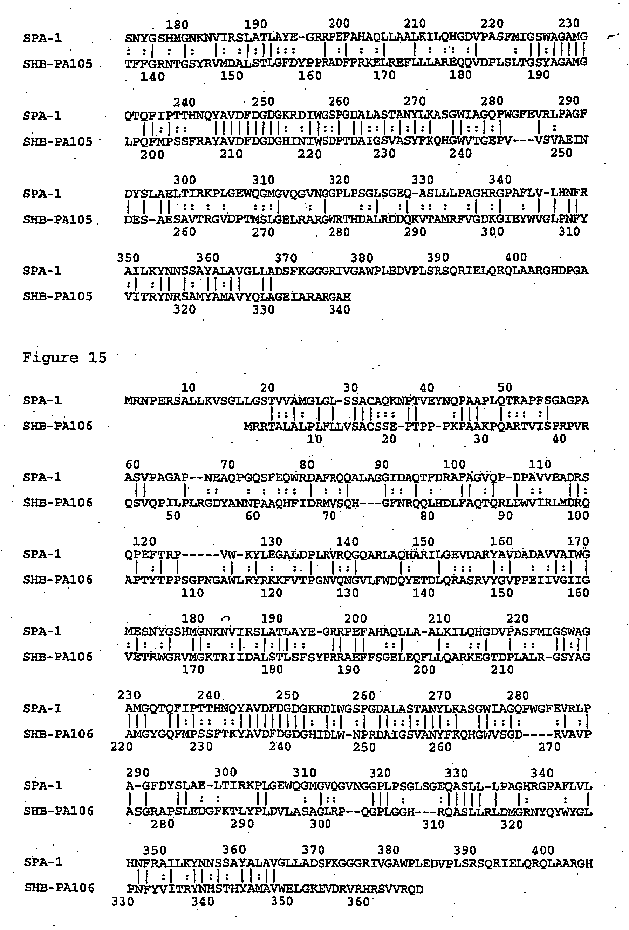

Genome analysis allowed the identification of 3 genes coding for proteins homologous to SPA-1. The sequences of each gene and protein are presented in Figures 7, 9, 11 and Figures 8, 10, 12 respectively. *SHB*-*PA104* (SEQ ID No: 8), SHB-PA105 (SEQ ID No: 10) and SHB-PA106 (SEQ ID No: 12) proteins present 49.4 % (over 389 aa; Figure 13), 33.2 % (over 361 aa; Figure 14) and 32.2 % (over 289 aa; Figure 15) identity with SPA-1 protein (448 aa) respectively. A paper presenting the 4 homologous proteins was published in January 2002 (Blackburn, N.T. and Clarke, A.J. (2002) Biochemistry, 41: 1001-1013). The paper describes these proteins as a family of lytic transglycosylases. Due to homologies with SPA-1, they may represent interesting, accessible vaccine candidates. Table 6 describes primers to amplify the three novel genes that can be overexpressed, purified and used as immunogens as for SPA-1.

**Table 6. Oligonucleotide primers for PCR amplification of new P. aeruginosa genes.**

| **Genes** | **Primers I.D.** | **Restriction site** | **Vector** | **Sequence** | **Sequence I.D. No.** |
|---|---|---|---|---|---|
| SHB-PA104 | PSEU446 | *Nde*I | pET19b | | 25 |
| SHB-PA104 | PSEU622 | *Bam*HI | pET19b | | 26 |
| SHB-PA105 | PSEU442 | *Nde*I | pET19b | | 27 |
| SHB-PA105 | PSEU443 | *Bam*HI | pET19b | | 28 |
| SHB-PA106 | PSEU438 | *Nde*I | pET19b | | 29 |
| SHB-PA106 | *PSEU638* | *Hind*III | pET19b | | 30 |

### Example 11. This example illustrates the method used for extracting lipids from bacterial cells.

Complex lipid mixtures were extracted from E. coli in order to generate liposome formulations from bacterial origin. To generate such complex lipid mixtures other bacterial species would have also been suitable such as: Neisseria spp, Haemophilus spp, Pseudomonas spp, bacteriodes spp, Legionella spp, Vibrio spp, Brucella spp, Bordetella spp, Campylobacter spp, Klebsiella spp, Salmonella spp, Shigella spp, Proteus spp, and **Yersinia** spp. Other species could also be used. The following method was used to generate the complex lipid mixtures used to generate the liposome formulations presented in Example 12.

Bacteria were grown overnight in BHI broth at 37 °C in presence of 8% CO₂ (175 rpm). Cells were collected by centrifugation and the pellet was suspended in 6.7 ml of methanol per gram of cells (wet weight). This bacterial suspension was sonicated in an ice bath twice using a Sonic dismembrator 500 (Fisher Scientific) with a microtip probe adjusted at 8. This suspension was then heated at 65°C for 30 min. After this incubation period, 2 volumes of chloroform were added to the suspension and agitated for 1 h at room temperature. The suspension was filtered through Whatman No. 4 filter. The filtrate was transferred in a Teflon tube and 0.2 volume of saline solution (NaCl 0.6% (w/v)) was then added. After centrifugation, the upper phase and the precipitate at the interface were discarded. The lower phase was extracted with one volume of chloroform:methanol:saline solution (3:48:47) at least four times or until there was no more precipitate at the interface. After the final extraction, the lower organic phase was dried in a rotatory evaporator (Rotavapor, Büchi, Switzerland). The dried phospholipids were stored at -80°C or resuspended in a solution of chloroform:methanol (2:1).

### Example 12. This example illustrates the incorporation of recombinant SPA-1 into different liposome formulations.

Liposomes were prepared using a dialysis method. Liposomes were prepared with different synthetic (see list 1 in this Example; Other lipids can be used and are described in Remington's on Pharmaceutical Sciences, 18th ed., 1990, Mack Publishing Co., Pennsylvania, p.390.) or bacterial phospholipids and/or cholesterol, which were combined at different ratios. Some liposome formulation were also prepared with the adjuvant monophosphoryl lipid A (MPLA, Avanti polar lipids, Alabaster, AL) at 600 µg/ml. SPA-1 protein was first precipitated in 90% ethanol (vol/vol) and denatured in 1 ml of PBS buffer containing 1% (wt/vol) of SDS (Sigma chemical) in PBS buffer, and heated at 100°C for 10 min. The solution was diluted with 1 ml of PBS buffer containing 15% (wt/vol) of n-octyl •-D-glucopyranoside (OG, Sigma) and incubated at room temperature for 3 h. Lipids were dissolved in a chloroform:methanol solution (2:1) in a round bottom glass flask and dried using a rotatory evaporator (Rotavapor, Buchi, Switzerland) to achieve an even film on the vessel. The above protein-detergent solution was then added to the lipid film and mixed gently until the film was dissolved. The solution after mixing was slightly opalescent in appearance. The solution was then extensively dialysed against PBS buffer (pH 7.4) to remove detergent and to induce liposome formation. After dialysis, the resulting milky solution was sequentially extruded through 1000, 400, 200, and 100 nm polycarbonate filters using a stainless steel extrusion device (Lipex Biomembranes, Vancouver, Canada). The unencapsulated proteins were removed by ultracentrifugation at 25 0000 x g for 1 h at 4ºC. The pellet was suspended with PBS buffer containing 0.3 M of sucrose. Vesicle size and homogeneity were evaluated by quasi-elastic light scattering with a submicron particles analyzer (model N4 Plus, Beckman Coulter). Using this apparatus, it was estimated that the liposome size in the different preparations was approximately 100 nm. All liposome preparations were sterilized by filtration through a 0.22-µm membrane and stored at -80ºC until used. The amount of recombinant protein incorporated in the liposome was estimated by MicroBCA (Pierce, Rockford, I11.) after phospholipid extraction of SPA-1-liposome preparations with chloroform:methanol solution (2:1) as described by Wessel and Flügge (Anal. Biochem. 1984, 138:141-143).

Gel filtration was used as an alternate method to induce the formation of SPA-1. liposome from the SPA-1-OG-SDS-lipids mixed micellar solution and to remove detergents. The SPA-1-OG-SDS-lipids solution was applied directly on top of a Sephadex G-50 (column size: 2 x 20cm, Pharmacia) or a P-6 (column size: 2 x 20cm, Bio Rad) size exclusion chromatography/desalting column and eluted with PBS buffer at a flow rate of 2.5 ml/min. Fractions containing both protein and lipids were pooled, extruded, centrifuged, and the vesicle *sizes* were evaluated as described above. All preparations were sterilized through a 0.22-µm membrane and stored at -80°C until used.

### List 1. Partial list on synthetic lipids used to prepare SPA-1-liposome preparations.

1,2-Dilauroyl-*sn*-Glycero-3-Phosphate (DLPA), Dimyristoyl-*sn-*Glycero-3-Phosphate (DMPA), 1,2-Dipalmitoyl-*sn*-Glycero-3-Phosphate (DPPA), 1,2-Distearoyl-*sn*-Glycero-3-Phosphate (DSPA), 1,2-Dioleoyl-*sn*-Glycero-3-Phosphate (DOPA), 1-Palmitoyl-2-Oleoyl-*sn*-Glycero-3-Phosphate (POPA), 1,2-Dilauroyl-*sn*-Glycero-3-Phosphocholine (DLPC), 1,2-Ditridecanoyl-*sn*-Glycero-3-Phosphocholine, 1,2-Dimyristoyl-*sn*-Glycero-3-Phosphocholine (DMPC), 1,2-Dipentadecanoyl-sn-Glycero-3-Phosphocholine, 1,2-Dipalmitoyl-*sn*-Glycero-3-Phosphocholine (DPPC), 1,2-Diheptadecanoyl-*sn*-Glycero-3-Phosphocholine, 1,2-Distearoyl-*sn-*Glycero-3-Phosphocholine (DSPC), 1,2-Dimyristoleoyl-*sn*-Glycero-3-Phosphocholine, 1,2-Dipalmitoleoyl-*sn*-Glycero-3-Phosphocholine, 1,2-Dioleoyl-sn-Glycero-3-Phosphocholine (DOPC), 1-Myristoyl-2-Palmitoyl-*sn*-Glycero-3-Phosphocholine, 1-Myristoy1-2-Stearoyl-*sn*-Glycero-3-Phosphocholine, 1-Palmitoyl-2-Myristoyl-*sn*-Glycero-3-Phosphocholine, 1-Palmitoyl-2-Stearoyl-*sn*-Glycero-3-Phosphocholine, 1-Palmitoyl-2-Oleoyl-*sn*-Glycero-3-Phosphocholine (POPC), 1-Palmitoyl-2-Linoleoyl-*sn*-Glycero-3-Phosphocholine, 1,2-Dilauroyl-sn-Glycero-3-Phosphoethanolamine (DLPE), 1,2-Dimyristoyl-*sn*-Glycero-3-Phosphoethanolamine (DMPE), 1,2-Dipalmitoyl-*sn*-Glycero-3-Phosphoethanolamine (DPPE), 1,2-Dipalmitoleoyl-*sn*-Glycero-3-Phosphoethanolamine, 1,2-Distearoyl-sn-Glycero-3-Phosphoethanolamine (DSPB), 1,2-Dioleoyl-*sn-*Glycero-3-Phosphoethanolamine (DOPE), 1-Palmitoyl-2-Oleoyl-*sn-*Glycero-3-Phosphoethanolamine (POPE), 1,2-Dilauroyl-*sn*-Glycero-3-[Phospho-*RAC*-(1-glycerol)] (DLPG), 1,2-Dimyristoyl-*sn*-Glycero-3-[Phospho-*RAC*-(1-glycerol)] (DMPG), 1,2-Dipalmitoyl-*sn*-Glycero-3-[Phospho-*RAC*-(1-glycerol)] (DPPG), 1,2-Distearoyl-*sn*-Glycero-3- [Phospno-*RAC*-(1-glycerol)] (DSPG), 1,2-Dioleoyl-*sn*-Glycero-3-[Phospho-*RAC*-(1-glycerol)] (DOPG), 1-Palmitoyl-2-Oleoyl-*sn-*Glycero-3-[Phospho-*RAC*-(1-glycerol)] (POPG), 1,2-Dilauroyl-*sn-*Glycero-3-[Phospho-L-Serine] (DLPS), 1,2-Dimyristoyl-*sn*-Glycero-3-[Phospho-L-serine] (DMPS)*,* 1,2-Dipalmitoyl-*sn*-Glycero-3-[Phospho-L-Serine] (DPPS), 1,2-Distearoyl-*sn*-Glycero-3-[Phospho-L-Serine] (DSPS), 1,2-Dioleoyl-*sn*-Glycero-3-[Phospho-L-Serine] (DOPS), 1-Palmitoyl-2-Oleoyl-*sn*-Glycero-3-[Phospho-L-Serine] (POPS).

### Example 13. This example illustrates the immunization of mice and rabbits with SPA-1-liposome formulations.

Groups of female BALB/c mice (Charles River Laboratories, St-Constant, Quebec, Canada) were immunized intramuscularly (IM) four times at two-week intervals with 20 µg of recombinant SPA-1 incorporated into different liposome reparations or, as control, with protein-free liposome formulations. Blood samples were collected from the orbital sinus prior to each immunization and two weeks after the last injection. The serum samples were stored at -20°C.

New Zealand White female rabbits (2.5kg, Charles River) were immunized IM three or four times at three-week intervals at several sites with 100 µg of recombinant SPA-1 protein incorporated in different liposome formulations. Serum samples were collected before each immunization and three weeks after the last injection. The serum samples were stored at -20ºC.

The application discloses the following embodiments
1. An isolated polynucleotide comprising a polynucleotide chosen from:
   (a) a polynucleotide encoding a polypeptide having at least 70%. identity to a second polypeptide comprising a sequence chosen from: SEQ ID NOS: 2, 4, 6, 8, 10, 12 or fragments or analogs thereof;
   (b) a polynucleotide encoding a polypeptide having at least 80% identity to a second polypeptide comprising a sequence chosen from: SEQ ID NOS: 2, 4, 6, 8, 10, 12 or fragments or analog thereof;
   (c) a polynucleotide encoding a polypeptide having at least 95% identity to a second polypeptide comprising a sequence chosen from: SEQ ID NOS: 2, 4, 6, 8, 10, 12 or fragments or analogs thereof;
   (d) a polynucleotide encoding a polypeptide comprising a sequence chosen from: SEQ ID NOS: 2, 4, 6, 8, 10, 12 or fragments or analogs thereof;
   (e) a polynucleotide encoding a polypeptide capable of raising antibodies haying binding specificity for a polypeptide. comprising a sequence chosen from: SEQ ID NOS: 2, 4, 6, 8, 10, 12 or fragments or analogs thereof;
   (f) a polynucleotide encoding an epitope bearing portion of a polypeptide comprising a sequence chose from SEQ ID NOS: 2, 4, 6, 8, 10, 12 or fragments or analogs thereof;
   (g) a polynucleotide comprising a sequence chosen from SEQ ID NOS: 1, 3, 5, 7, 9, 11 or fragments or analogs thereof;
   (h) a polynucleotide that is complementary to a polynucleotide in (a), (b), (c), (d), (e), (f) or (g).
2. An isolated polynucleotide comprising a polynucleotide chosen from:
   (a) a polynucleotide encoding a polypeptide having at least 70% identity to a second polypeptide comprising a sequence chosen from: SEQ ID NOS: 2, 4, 6, 8, 10 or 12;
   (b) a polynucleotide encoding a polypeptide having at least 80% identity to a second polypeptide comprising a sequence chosen from: SEQ ID NOS: 2, 4, 6, 8, 10 or 12;
   (c) a polynucleotide encoding a polypeptide having at least 95% identity to a second polypeptide comprising a sequence chosen from: SEQ ID NOS: 2, 4, 6, 8, 10 or 12;
   (d) a polynucleotide encoding a polypeptide comprising a sequence chosen from: SEQ ID NOS: 2, 4, 6, 8, 10 or 12;
   (e) a polynucleotide encoding a polypeptide capable of raising antibodies having binding specificity for a polypeptide comprising a sequence chosen from: SEQ ID NOS: 2, 4, 6, 8, 10 or 12;
   (f) a polynucleotide encoding an epitope bearing portion of a polypeptide comprising a sequence chosen from SEQ ID NOS: 2, 4, 6, 8, 10 or 12;
   (g) a polynucleotide comprising a sequence chosen from SEQ ID NOS: 1, 3, 5, 7, 9 or 11;
   (h) a polynucleotide that is complementary to a polynucleotide in (a), (b), (c), (d), (e), (f) or (g).
3. The polynucleotide of item 1, wherein said polynucleotide is DNA.
4. The polynucleotide of item 2, wherein said polynucleotide is DNA.
5. The polynucleotide of item 1, wherein said polynucleotide is RNA.
6. The polynucleotide of item 2, wherein said polynucleotide is RNA.
7. An isolated polynucleotide that hybridizes under stringent conditions to either
   (a) a DNA sequence encoding a polypeptide or
   (b) the complement of a DNA sequence encoding a polypeptide; wherein said polypeptide comprises a sequence chosen from SEQ ID NOS: 2, 4, 6, 8, 10, 12 or fragments or analogs thereof.
8. The polynucleotide of item 1 that hybridizes under stringent conditions to either
   (a) a DNA sequence encoding a polypeptide or
   (b) the complement of a DNA sequence encoding a polypeptide; wherein said polypeptide comprises a sequence chosen from SEQ ID NOS: 2, 4, 6, 8, 10, 12 or fragments or analogs thereof.
9. The polynucleotide of item 2 that hybridizes under stringent conditions to either
   (a) a DNA sequence encoding a polypeptide or
   (b) the complement of a DNA sequence encoding a polypeptide; wherein said polypeptide comprises a sequence chosen from SEQ ID NOS: 2, 4, 6, 8, 10 or 12.
10. The polynucleotide of item 1 that hybridizes under stringent conditions to either
   (a) a DNA sequence encoding a polypeptide or
   (b) the complement of a DNA sequence encoding a polypeptide; wherein said polypeptide comprises at least 10 contiguous amino acid residues from a polypeptide comprising a sequence chosen from SEQ ID NOS: 2, 4, 6, 8, 10, 12 or fragments or analogs thereof.
11. The polynucleotide of item 2 that hybridizes under stringent conditions to either
   (a) a DNA sequence encoding a polypeptide or
   (b) the complement of a DNA sequence encoding a polypeptide; wherein said polypeptide comprises at least 10 contiguous amino acid residues from a polypeptide comprising a sequence chosen from SEQ ID NOS: 2, 4, 6, 8, 10 or 12.
12. An isolated polynucleotide having a sequence comprising SEQ ID NOs: 1, 3, 5, 7, 9, 11 or fragments or analogs thereof.
13. An isolated polynucleotide having a sequence comprising SEQ ID NOs: 1, 3, 5, 7, 9 or 11.
14. A vector comprising the polynucleotide of item 1, wherein said polynucleotide is operably linked to an expression control region.
15. A vector comprising the polynucleotide of item 2, wherein said polynucleotide is operably linked to an expression control region.
16. A host cell transfected with the vector of item 14.
17. A host cell transfected with the vector of item 15.
18. A process for producing a polypeptide comprising culturing a host cell according to item 16 under conditions suitable for expression of said polypeptide.
19. A process for producing a polypeptide comprising culturing a host cell according to item 17 under condition suitable for expression of said polypeptide.
20. An isolated polypeptide comprising a polypeptide chosen from:
   (a) a polypeptide having at least 70% identity to a second polypeptide comprising a sequence chosen from SEQ ID NOS: 2, 4, 6, 8, 10, 12 or fragments or analogs thereof;
   (b) a polypeptide having at least 80% identity to a second polypeptide comprising a sequence chosen from SEQ ID NOS: 2, 4, 6, 8, 10, 12 or fragments or analogs thereof;
   (c) a polypeptide having at least 95% identity to a second polypeptide comprising a sequence chosen from SEQ ID NOS: 2, 4, 6, 8, 10, 12 or fragments or analogs thereof;
   (d) a polypeptide comprising a sequence chosen from SEQ ID NOS: 2, 4, 6, 8, 10, 12 or fragments or analogs thereof;
   (e) a polypeptide capable of raising antibodies having binding specificity for a polypeptide comprising a sequence chosen from SEQ ID NOS: 2, 4, 6, 8, 10, 12 or fragments or analogs thereof;
   (f) an epitope bearing portion of a polypeptide comprising a sequence chosen from SEQ ID NOS: 2, 4, 6, 8, 10, 12 or fragments or analogs thereof;
   (g) the polypeptide of (a), (b), (c), (d), (e) or (f) wherein the N-terminal Met residue is deleted;
   (h) the polypeptide of (a), (b), (c), (d), (e), (f) or (g) wherein the secretory amino acid sequence is deleted.
21. An isolated polypeptide comprising a polypeptide chosen from:
   (a) a polypeptide having at least 70% identity to a second polypeptide comprising a sequence chosen from SEQ ID NOS: 2, 4, 6, 8, 10 or 12;
   (b) a polypeptide having at least 80% identity to a second polypeptide comprising a sequence chosen from SEQ ID NOS: 2, 4, 6, 8, 10 or 12;
   (c) a polypeptide having at least 95% identity to a second polypeptide comprising a sequence chosen from SEQ ID NOS: 2, 4, 6, 8, 10 or 12;
   (d) a polypeptide comprising a sequence chosen from SEQ ID NOS: 2, 4, 6, 8, 10 or 12;
   (e) a polypeptide capable of raising antibodies having binding specificity for a polypeptide comprising a sequence chosen from SEQ ID NOS: 2, 4, 6, 8, 10 or 12;
   (f) an epitope bearing portion of a polypeptide comprising a sequence chosen from SEQ ID NOS: 2, 4, 6, 8, 10 or 12;
   (g) the polypeptide of (a), (b), (c), (d), (e) or (f) wherein the N-terminal Met residue is deleted;
22. The polypeptide of (a), (b), (c), (d), (e), (f) or (g) wherein the secretory amino acid sequence is deleted.
23. A chimeric polypeptide comprising two or more polypeptides having a sequence chosen from SEQ ID NOS: 2, 4, 6, 8, 10, 12 or fragments or analogs thereof; provided that the polypeptides are linked as to formed a chimeric polypeptide.
24. A chimeric polypeptide comprising two or more polypeptides having a sequence chosen from SEQ ID NOS: 2, 4, 6, 8, 10 or 12 provided that the polypeptides are linked as to formed a chimeric polypeptide.
25. A pharmaceutical composition comprising a polypeptide according to any one of items 20 to 24 and a pharmaceutically acceptable carrier, diluent or adjuvant.
26. A pharmaceutical composition comprising a polypeptide according to any one of items 20 to 24 and a liposome.
27. A method for therapeutic or prophylactic treatment of *Pseudomonas aeruginosa bacterial infection in a* host susceptible to Pseudomonas aeruginosa infection comprising administering to said host a therapeutic or prophylactic amount of a composition according to item 25.
28. A method for therapeutic or prophylactic treatment of pneumonia, bacteremia, chronical infection or septicemia, in a host susceptible to Pseudomonas aeruginosa infection, comprising administering to said host a therapeutic or prophylactic amount of a composition according to item 25.
29. A method for therapeutic or prophylactic treatment of pneumonia, bacteremia, chronical infection or septicemia, in a host susceptible to Pseudomonas aeruginosa infection, comprising administering to said host a therapeutic or prophylactic amount of a composition according to item 26.
30. A method for diagnostic of Pseudomonas infection in an host susceptible to Pseudomonas infection comprising
   (a) obtaining a biological sample from a host;
   (b) incubating an antibody or fragment thereof reactive with a Pseudomonas polypeptide of the invention with the biological sample to form a mixture; and
   (c) detecting specifically bound antibody or bound fragment in the mixture which indicates the presence of Pseudomonas.
31. A method for diagnostic of Pseudomonas infection in an host susceptible to Pseudomonas infection comprising
   (a) obtaining a biological sample from a host;
   (b) incubating one or more Pseudomanas polypeptides of the invention or fragments thereof with the biological sample to form a mixture; and
   (c) detecting specifically bound antigen or bound fragment in the mixture which indicates the presence of antibody specific to Pseudomonas.
32. A method for treatment of Pseudomonas infection using an antibody directed to a polypeptide according to any one of items 20 to 24.
33. Use of the pharmaceutical composition according to item 25 in the manufacture of a medicament for the prophylactic or therapeutic treatment of Pseudomonas infection.
34. Use of the pharmaceutical composition according to item 26 in the manufacture of a medicament for the prophylactic or therapeutic treatment of Pseudomonas infection.
35. Kit comprising a polypeptide according to any one of items 20 to 24 for detection or diagnosis of Pseudomonas infection.

## Claims

1. A pharmaceutical composition comprising a pharmaceutically acceptable carrier or diluent and an isolated polypeptide chosen from:
(a) an isolated polypeptide comprising an amino acid sequence at least 90% identical to the amino acid sequence set forth in SEQ ID NO:4;
(b) an isolated polypeptide comprising an amino acid sequence at least 95% identical to the amino acid sequence set forth in SEQ ID NO:4;
(c) an isolated polypeptide comprising the amino acid sequence set forth in SEQ ID NO: 4;
(d) an isolated polypeptide comprising a polypeptide fragment wherein the fragment comprises at least 20 contiguous amino acids of the amino acid sequence set forth in SEQ ID NO:4;
(e) the isolated polypeptide of (c) from which the secretory amino acid sequence is deleted; and
(f) an isolated polypeptide from which the secretory amino acid sequence is deleted comprising an amino acid sequence at least 90% identical to the amino acid sequence of the non-underlined portion of the sequence of Figure 4;
wherein the isolated polypeptide is capable of eliciting antibodies that specifically bind to a polypeptide consisting of the amino acid sequence set forth in SEQ ID NO:4 and is capable of eliciting an immune response to *Pseudomonas aeruginosa.*

2. The pharmaceutical composition according to claim 1 wherein the isolated polypeptide is conjugated or coupled to a carrier protein.

3. The pharmaceutical composition according to claim 1 or claim 2 further comprising a pharmaceutically acceptable adjuvant.

4. The pharmaceutical composition according to claim 1 or claim 2 wherein the isolated polypeptide is associated with a liposome.

5. The pharmaceutical composition according to claim 1 or claim 2 wherein the isolated polypeptide is produced recombinantly by a method comprising culturing a host cell transfected with a vector that comprises a polynucleotide encoding the isolated polypeptide, wherein the polynucleotide is operable linked to an expression control region.

6. The pharmaceutical composition according to claim 1 wherein the isolated polypeptide of (a), (b), (c), (d), (e) or (f) is linked to one or more further antigenic polypeptides to form a chimeric polypeptide comprising two or more antigenic polypeptides having a sequence chosen from SEQ ID NOS: 2, 4, 6, 8, 10 or 12 or fragments thereof wherein each of the two or more antigenic polypeptide fragments comprises at least 20 contiguous amino acids of the amino acid sequence set forth in SEQ ID NO:2, 4, 6, 8, 10 or 12.

7. The pharmaceutical composition of claim 6 comprising a pharmaceutically acceptable carrier, diluent, adjuvant or liposome.

8. Kit for detection or diagnosis of a *Pseudomonas aeruginosa* infection, said kit comprising:
(a) the chimeric polypeptide according to claim 6;
(b) an isolated polypeptide comprising an amino acid sequence at least 90% identical to the amino acid sequence set forth in SEQ ID NO:4;
(c) an isolated polypeptide comprising an amino acid sequence at least 95% identical to the amino acid sequence set forth in SEQ ID NO:4;
(d) an isolated polypeptide comprising the amino acid sequence set forth in SEQ ID NO:4;
(e) an isolated polypeptide comprising a polypeptide fragment wherein the fragment comprises at least 20 contiguous amino acids of the amino acid sequence set forth in SEQ ID NO:4; and
(g) the isolated polypeptide of (b), (c), or (d), from which the signal peptide sequence is deleted,
wherein the isolated polypeptide is capable of eliciting antibodies that specifically bind to a polypeptide consisting of the amino acid sequence set forth in SEQ ID NO:4.

9. The pharmaceutical composition according to any one of claims 1-7 for use in the prophylactic or therapeutic treatment of a *Pseudomonas aeruginosa* infection.

10. Use of the pharmaceutical composition according to any one of claims 1-7 for the manufacture of a vaccine for the prophylactic or therapeutic treatment of a *Pseudomonas aeruginosa* infection.

## Patentansprüche

1. Pharmazeutische Zusammensetzung, umfassend ein pharmazeutisch annehmbares Träger- oder Verdünnungsmittel und ein isoliertes Polypeptid ausgewählt aus:
(a) einem isolierten Polypeptid, umfassend eine Aminosäuresequenz, die mindestens 90 % identisch mit der Aminosäuresequenz ist, die in SEQ ID NO: 4 dargestellt ist;
(b) einem isolierten Polypeptid, umfassend eine Aminosäuresequenz, die mindestens 95 % identisch mit der Aminosäuresequenz ist, die in SEQ ID NO: 4 dargestellt ist;
(c) einem isolierten Polypeptid, umfassend die Aminosäuresequenz, die in SEQ ID NO: 4 dargestellt ist;
(d) einem isolierten Polypeptid, umfassend ein Polypeptidfragment, wobei das Fragment mindestens 20 aufeinanderfolgende Aminosäuren der Aminosäuresequenz umfasst, die in SEQ ID NO: 4 dargestellt ist;
(e) dem isolierten Polypeptid gemäß (c), von dem die sekretorische Aminosäuresequenz deletiert ist; und
(f) einem isolierten Polypeptid, von dem die sekretorische Aminosäuresequenz deletiert ist, umfassend eine Aminosäuresequenz, die mindestens 90 % identisch mit der Aminosäuresequenz des nicht unterstrichenen Bereichs der Sequenz von Figur 4 ist;
wobei das isolierte Polypeptid in der Lage ist, Antikörper hervorzurufen, die spezifisch an ein Polypeptid binden, das aus der Aminosäuresequenz besteht, die in SEQ ID NO: 4 dargestellt ist und in der Lage ist, eine Immunantwort auf *Pseudomonas aeruginosa* hervorzurufen.

2. Pharmazeutische Zusammensetzung gemäß Anspruch 1, wobei das isolierte Polypeptid an ein Trägerprotein konjugiert oder gekoppelt ist.

3. Pharmazeutische Zusammensetzung gemäß Anspruch 1 oder Anspruch 2, weiterhin umfassen ein pharmazeutisch annehmbares Adjuvanz.

4. Pharmazeutische Zusammensetzung gemäß Anspruch 1 oder Anspruch 2, wobei das isolierte Polypeptid mit einem Liposom assoziiert ist.

5. Pharmazeutische Zusammensetzung gemäß Anspruch 1 oder Anspruch 2, wobei das isolierte Polypeptid rekombinant durch ein Verfahren hergestellt wird, das das Kultivieren einer Wirtszelle umfasst, die mit einem Vektor transfiziert ist, der ein Polynukleotid kodierend das isolierte Polypeptid umfasst, wobei das Polynukleotid operabel an eine Expressionskontrollregion geknüpft ist.

6. Pharmazeutische Zusammensetzung gemäß Anspruch 1, wobei das isolierte Polypeptid gemäß (a), (b), (c), (d), (e) oder (f) an ein oder mehrere weitere antigene Polypeptide geknüpft ist, um ein chimäres Polypeptid zu bilden, das zwei oder mehr antigene Polypeptide umfasst und eine Sequenz hat, die aus SEQ ID NOS: 2, 4, 6, 8, 10 oder 12 oder Fragmenten davon ausgewählt wird, wobei jedes der zwei oder mehr antigenen Polypeptidfragmente mindestens 20 aufeinanderfolgende Aminosäuren der Aminosäuresequenz hat, die in SEQ ID NO: 2, 4, 6, 8, 10 oder 12 dargestellt ist.

7. Pharmazeutische Zusammensetzung gemäß Anspruch 6, umfassend ein pharmazeutisch annehmbares Trägermittel, Verdünnungsmittel, Adjuvanz oder Liposom.

8. Kit zum Nachweisen oder zur Diagnose einer *Pseudomonas aeruginosa*-Infektion, wobei das Kit umfasst:
(a) das chimäre Polypeptid gemäß Anspruch 6;
(b) ein isoliertes Polypeptid, umfassend eine Aminosäuresequenz, die mindestens 90 % identisch mit der Aminosäuresequenz ist, die in SEQ ID NO: 4 dargestellt ist;
(c) ein isoliertes Polypeptid, umfassend eine Aminosäuresequenz, die mindestens 95 % identisch mit der Aminosäuresequenz ist, die in SEQ ID NO: 4 dargestellt ist;
(d) ein isoliertes Polypeptid, umfassend die Aminosäuresequenz, die in SEQ ID NO: 4 dargestellt ist;
(e) ein isoliertes Polypeptid, umfassend ein Polypeptidfragment, wobei das Fragment mindestens 20 aufeinanderfolgende Aminosäuren der Aminosäuresequenz umfasst, die in SEQ ID NO: 4 dargestellt ist; und
(g) das isolierte Polypeptid gemäß (b), (c) oder (d), von dem die Signalpeptidsequenz deletiert ist,
wobei das isolierte Polypeptid in der Lage ist, Antikörper hervorzurufen, die spezifisch an ein Polypeptid binden, das aus der Aminosäuresequenz besteht, die in SEQ ID NO: 4 dargestellt ist.

9. Pharmazeutische Zusammensetzung gemäß einem der Ansprüche 1 bis 7 zur Verwendung bei der prophylaktischen oder therapeutischen Behandlung einer *Pseudomonas aeruginosa*-Infektion.

10. Verwendung der pharmazeutischen Zusammensetzung gemäß einem der Ansprüche 1 bis 7 zur Herstellung eines Impfstoffs zur prophylaktischen oder therapeutischen Behandlung einer *Pseudomonas aeruginosa*-Infektion*.*

## Revendications

1. Composition pharmaceutique comprenant un support ou un diluant pharmaceutiquement acceptable et un polypeptide isolé choisi parmi :
(a) un polypeptide isolé comprenant une séquence d'acides aminés au moins identique à 90 % avec la séquence d'acides aminés représentée par SEQ ID NO : 4 ;
(b) un polypeptide isolé comprenant une séquence d'acides aminés au moins identique à 95 % avec la séquence d'acides aminés représentée par SEQ ID NO : 4 ;
(c) un polypeptide isolé comprenant la séquence d'acides aminés représentée par SEQ ID NO : 4 ;
(d) un polypeptide isolé comprenant un fragment de polypeptide où le fragment comprend au moins 20 acides aminés consécutifs de la séquence d'acides aminés représentée par SEQ ID NO : 4 ;
(e) le polypeptide isolé de (c) à partir duquel la séquence d'acides aminés sécrétoire est délétée ; et
(f) un polypeptide isolé à partir duquel la séquence d'acides aminés sécrétoire est délétée comprenant une séquence d'acides aminés au moins identiques à 90 % avec la séquence d'acides aminés de la partie non soulignée de la séquence de la figure 4 ;
où le polypeptide isolé est capable de déclencher des anticorps qui se lient spécifiquement à un polypeptide constitué de la séquence d'acides aminés représentée par SEQ ID NO : 4 et est capable de déclencher une réponse immunitaire contre *Pseudomonas aeruginosa.*

2. Composition pharmaceutique selon la revendication 1, dans laquelle le polypeptide isolé est conjugué ou couplé à une protéine de transport.

3. Composition pharmaceutique selon la revendication 1 ou la revendication 2, comprenant en outre un adjuvant pharmaceutiquement acceptable.

4. Composition pharmaceutique selon la revendication 1 ou la revendication 2, dans laquelle le polypeptide isolé est associé à un liposome.

5. Composition pharmaceutique selon la revendication 1 ou la revendication 2, dans laquelle le polypeptide isolé est produit de manière recombinante par un procédé comprenant la culture d'une cellule hôte transfectée avec un vecteur qui comprend un polynucléotide codant pour le polypeptide isolé, où le polynucléotide est lié de manière fonctionnelle à une région de contrôle de l'expression.

6. Composition pharmaceutique selon la revendication 1, dans laquelle le polypeptide isolé de (a), (b), (c), (d), (e) ou (f) est lié à un ou plusieurs autres polypeptides antigéniques pour former un polypeptide chimérique comprenant deux polypeptides antigéniques ou plus ayant une séquence choisie parmi SEQ ID NO : 2, 4, 6, 8, 10 ou 12 ou des fragments de celles-ci où chacun des deux fragments polypeptidiques antigéniques ou plus comprend au moins 20 acides aminés consécutifs de la séquence d'acides aminés représentée par SEQ ID NO : 2, 4, 6, 8, 10 ou 12.

7. Composition pharmaceutique selon la revendication 6, comprenant un support, un diluant, un adjuvant ou un liposome pharmaceutiquement acceptable.

8. Kit de détection ou de diagnostic d'une infection à *Pseudomonas aeruginosa,* ledit kit comprenant :
(a) le polypeptide chimérique selon la revendication 6 ;
(b) un polypeptide isolé comprenant une séquence d'acides aminés au moins identique à 90 % avec la séquence d'acides aminés représentée par SEQ ID NO : 4 ;
(c) un polypeptide isolé comprenant une séquence d'acides aminés au moins identique à 95 % avec la séquence d'acides aminés représentée par SEQ ID NO : 4 ;
(d) un polypeptide isolé comprenant la séquence d'acides aminés représentée par SEQ ID NO : 4 ;
(e) un polypeptide isolé comprenant un fragment de polypeptide où le fragment comprend au moins 20 acides aminés consécutifs de la séquence d'acides aminés représentée par SEQ ID NO : 4 ; et
(g) le polypeptide isolé de (b), (c) ou (d), à partir duquel la séquence du peptide signal est délétée,
où le polypeptide isolé est capable de déclencher des anticorps qui se lient spécifiquement à un polypeptide constitué de la séquence d'acides aminés représentée par SEQ ID NO : 4.

9. Composition pharmaceutique selon l'une quelconque des revendications 1 à 7 pour une utilisation dans le traitement prophylactique ou thérapeutique d'une infection à *Pseudomonas aeruginosa.*

10. Utilisation de la composition pharmaceutique selon l'une quelconque des revendications 1 à 7, pour la fabrication d'un vaccin destiné au traitement prophylactique ou thérapeutique d'une infection à *Pseudomonas aeruginosa.*


### SEQUENCE LISTING

<110> ID Biomedical Corporation
<120> Polypeptides of Pseudomonas Aeruginosa
<130> K1952 EP/1
<140> PCT/CA02/01740
   <141> 2002-11-13
<150> US 60/331,221
   <151> 2001-11-13
<160> 36
<170> PatentIn version 3.1
<210> 1
   <211> 1347
   <212> DNA
   <213> Pseudomonas aeruginosa
<400> 1
   atgcgtaacc ccgaacgatc cgccctgctg aaggtgagcg ggctgctggg cagcaccgtc      60
   gtcgccatgg ggcttggcct ctccagcgcc tgcgcgcaga agaatccgac agtcgaatac     120
   aaccagcctg ccgctcccct gcagaccaag gcgcccttct ccggcgccgg cccggccgcc     180
   tcggtgcccg ctggcgcgcc gaacgaggcc cagcctgggc aaagcttcga acagtggcgc     240
   gacgccttcc gtcaacaggc gctggccggt ggaatcgatg cgcagacctt cgatcgcgcc     300
   ttcgccggcg tccagcccga tcccgccgtg gtcgaagcag accgcagcca gccggaattc     360
   acccgaccgg tatggaagta cctggaaggc gccctcgatc cgctgcgcgt tcgccagggc     420
   caggcgcgcc tggcgcagca tgcgcgcatc ctcggcgaag tcgacgcgcg ctatgcggtg     480
   gatgcggatg cggtggtggc gatctggggc atggagagca actacggttc gcacatgggc     540
   aacaagaacg tgatccgctc cctggcgacc ctcgcctatg aaggacgccg cccggaattc     600
   gcccacgccc agttgctcgc cgccctgaag attctccagc acggcgacgt tccggcctcc     660
   ttcatgatcg gctcctgggc cggcgccatg ggccagaccc agttcatccc gaccacccac     720
   aaccagtatg ccgtggactt cgacggcgac ggcaagcgtg acatctgggg ctcgcccggc     780
   gacgccctgg cctccaccgc caactacctg aaagcctccg gctggatcgc cggacaaccc     840
   tggggtttcg aagtccgcct gccggcaggc ttcgactatt ccctggcgga actcaccatc     900
   cgcaagcccc tgggcgaatg gcaagggatg ggcgtacaag gcgtcaacgg cggccccctg    960
   ccctccggac tctccggcga acaggcctcg ctgctgctgc cggccgggca ccgcggcccg    1020
   gccttcctgg tgctgcacaa cttccgcgcc atcctcaagt acaacaactc cagcgcctac    1080
   gccctggccg tcggcctgct cgccgacagc ttcaagggcg gcggccggat agtcggcgcc    1140
   tggccgctgg aggatgttcc gctgagccgc tcgcagcgca tcgagctgca acggcaactg    1200
   gccgcccgcg gacacgatcc gggcgcggtg gatggcatca tcggcgccaa tacgcgcaag    1260
   gcgatccgcg cctgccagca ggagttcggc tggccggcgg acggctatcc gaccccggcg    1320
   ctgctcgacc gcctgcggac gccatag                                        1347
<210> 2
   <211> 448
   <212> PRT
   <213> Pseudomonas aeruginosa
<400> 2
   Met Arg Asn Pro Glu Arg Ser Ala Leu Leu Lys Val Ser Gly Leu Leu
   1               5                   10                  15
   Gly Ser Thr Val Val Ala Met Gly Leu Gly Leu Ser Ser Ala Cys Ala
               20                  25                  30
   Gln Lys Asn Pro Thr Val Glu Tyr Asn Gln Pro Ala Ala Pro Leu Gln
           35                  40                  45
   Thr Lys Ala Pro Phe Ser Gly Ala Gly Pro Ala Ala Ser Val Pro Ala
       50                  55                  60
   Gly Ala Pro Asn Glu Ala Gln Pro Gly Gln Ser Phe Glu Gln Trp Arg
   65                  70                  75                  80
   Asp Ala Phe Arg Gln Gln Ala Leu Ala Gly Gly Ile Asp Ala Gln Thr
                   85                  90                  95
   Phe Asp Arg Ala Phe Ala Gly Val Gln Pro Asp Pro Ala Val Val Glu
               100                 105                 110
   Ala Asp Arg Ser Gln Pro Glu Phe Thr Arg Pro Val Trp Lys Tyr Leu
           115                 120                 125
   Glu Gly Ala Leu Asp Pro Leu Arg Val Arg Gln Gly Gln Ala Arg Leu
   130 135 140
   Ala Gln His Ala Arg Ile Leu Gly Glu Val Asp Ala Arg Tyr Ala Val
   145 150 155 160
   Asp Ala Asp Ala Val Val Ala Ile Trp Gly Met Glu Ser Asn Tyr Gly
   165 170 175
   Ser His Met Gly Asn Lys Asn Val Ile Arg Ser Leu Ala Thr Leu Ala
   180 185 190
   Tyr Glu Gly Arg Arg Pro Glu Phe Ala His Ala Gln Leu Leu Ala Ala
   195 200 205
   Leu Lys Ile Leu Gln His Gly Asp Val Pro Ala Ser Phe Met Ile Gly
   210 215 220
   Ser Trp Ala Gly Ala Met Gly Gln Thr Gln Phe Ile Pro Thr Thr His
   225 230 235 240
   Asn Gln Tyr Ala Val Asp Phe Asp Gly Asp Gly Lys Arg Asp Ile Trp
   245 250 255
   Gly Ser Pro Gly Asp Ala Leu Ala Ser Thr Ala Asn Tyr Leu Lys Ala
   260 265 270
   Ser Gly Trp Ile Ala Gly Gln Pro Trp Gly Phe Glu Val Arg Leu Pro
   275 280 285
   Ala Gly Phe Asp Tyr Ser Leu Ala Glu Leu Thr Ile Arg Lys Pro Leu
   290 295 300
   Gly Glu Trp Gln Gly Met Gly Val Gln Gly Val Asn Gly Gly Pro Leu
   305 310 315 320
   Pro Ser Gly Leu Ser Gly Glu Gln Ala Ser Leu Leu Leu Pro Ala Gly
   325 330 335
   His Arg Gly Pro Ala Phe Leu Val Leu His Asn Phe Arg Ala Ile Leu
   340 345 350
   Lys Tyr Asn Asn Ser Ser Ala Tyr Ala Leu Ala Val Gly Leu Leu Ala
   355 360 365
   Asp Ser Phe Lys Gly Gly Gly Arg Ile Val Gly Ala Trp Pro Leu Glu
   370 375 380
   Asp Val Pro Leu Ser Arg Ser Gln Arg Ile Glu Leu Gln Arg Gln Leu
   385 390 395 400
   Ala Ala Arg Gly His Asp Pro Gly Ala Val Asp Gly Ile Ile Gly Ala
   405 410 415
   Asn Thr Arg Lys Ala Ile Arg Ala Cys Gln Gln Glu Phe Gly Trp Pro
   420 425 430
   Ala Asp Gly Tyr Pro Thr Pro Ala Leu Leu Asp Arg Leu Arg Thr Pro
   435 440 445
<210> 3
   <211> 624
   <212> DNA
   <213> Pseudomonas aeruginosa
<400> 3
   atgaaacgta tcctgaccag cgccgcgctg atcggtatga ccaccctgct ggccgcctgc    60
   ggcttccaac tgcgcggcct gggcgatgcg caattcgcgc tcaaggaaat cgacgtgtcc   120
   gcgcgcaacg cctacggccc gaccgtgcgc gaactgaagg aaaccctgga aaacagcggc   180
   gtgaaggtca ccagcaacgc gccctaccac ctggtgctgg tccgcgagga caaccagcag   240
   cgcaccgtca gctacaccgg ttccgcgcgc ggcgcggagt tcgagctgac caacacgatc   300
   aactacgaga tcgtcggcgc caacgacctg gtcctgatga gcaaccaggt acaggtgcag   360
   aaggtctacg tgcacgacga aaacaacctg atcggttccg accaggaagc cgcgcagctg   420
   cgcagcgaga tgcggcgcga cctgatccag cagttgtcca tgcgcctcca ggcgctgacc   480
   ccggcgcaac tcgacgaagc ccagcgccag gcagaagcca aggccaaggc ggaagccgaa   540
   gccctgcgcg ccgccgacga ggcggagcgc cagcgccgcg ccgccgagcc gcagcagtcg   600
   ccgatcgagt tccccacccc gtga                                          624
<210> 4
   <211> 207
   <212> PRT
   <213> Pseudomonas aeruginosa
<400> 4
   Met Lys Arg Ile Leu Thr Ser Ala Ala Leu Ile Gly Met Thr Thr Leu
   1 5 10 15
   Leu Ala Ala Cys Gly Phe Gln Leu Arg Gly Leu Gly Asp Ala Gln Phe
   20 25 30
   Ala Leu Lys Glu Ile Asp Val Ser Ala Arg Asn Ala Tyr Gly Pro Thr
   35 40 45
   Val Arg Glu Leu Lys Glu Thr Leu Glu Asn Ser Gly Val Lys Val Thr
   50 55 60
   Ser Asn Ala Pro Tyr His Leu Val Leu Val Arg Glu Asp Asn Gln Gln
   65 70 75 80
   Arg Thr Val Ser Tyr Thr Gly Ser Ala Arg Gly Ala Glu Phe Glu Leu
   85 90 95
   Thr Asn Thr Ile Asn Tyr Glu Ile Val Gly Ala Asn Asp Leu Val Leu
   100 105 110
   Met Ser Asn Gln Val Gln Val Gln Lys Val Tyr Val His Asp Glu Asn
   115 120 125
   Asn Leu Ile Gly Ser Asp Gln Glu Ala Ala Gln Leu Arg Ser Glu Met
   130 135 140
   Arg Arg Asp Leu Ile Gln Gln Leu Ser Met Arg Leu Gln Ala Leu Thr
   145 150 155 160
   Pro Ala Gln Leu Asp Glu Ala Gln Arg Gln Ala Glu Ala Lys Ala Lys
   165 170 175
   Ala Glu Ala Glu Ala Leu Arg Ala Ala Asp Glu Ala Glu Arg Gln Arg
   180 185 190
   Arg Ala Ala Glu Pro Gln Gln Ser Pro Ile Glu Phe Pro Thr Pro
   195 200 205
<210> 5
   <211> 1143
   <212> DNA
   <213> Pseudomonas aeruginosa
<400> 5
   atggtgcaat ggaaacacgc ggcgctgctc gccctggccc tggcggtcgt gggttgcagc 60
   agcaacagca agaaggaact cccgcccgcc gaactgaccg acttcaaaga ggaagtcgtg 120
   ttgagcaagc agtggagccg ctcggtcggt gatggtcagg gcgacctgta caacctgctc 180
   gaaccggccg tcgatggttc caccatctac gccgcgtccg ccgaagggcg ggtgatggcg 240
   atccagcgcg agaccggcga cgtgctctgg aagaaggacc tggaacgtcc ggtttccggc 300
   ggtgtcggcg ttggctacgg cctggtgctg gtgggtaccc tgcgcggtga cgtgatcgcc 360
   ctcgacgaag ccaccggcaa gaagaagtgg accaagcgag tcaacagcga agtgctgtcg 420
   gcgccggcca ccaatggcga cgtggtggtg gtgcagaccc aggacgacaa gctgatcggc    480
   ctcgatgcgg ccagcggcga ccagcgctgg atctacgaaa gcaccgtgcc ggtgctgacc    540
   ctgcgcggca ccggcgcgcc gctgattgcc ggcaacatgg ccctggctgg cctggccagc    600
   ggcaaggtag tggcggtcga cgtacagcgc ggcctgccga tctgggagca gcgggtagcg    660
   attccccagg ggcgttccga actggatcgc gtggtggaca tcgacggcgg cctcctgctg    720
   tccggcgaca ccctctacgt ggtcagctac cagggccgtg ccgcggcgct ggacgtgaac    780
   agcggccgcc tgctctggca gcgcgaagcg tcgagctacg tcggcgtcgc cgaaggcttc    840
   ggcaatatct acgtcagcca ggccagcggt tcggtggaag gcctggactc gcgcggcgct    900
   tcttcgctgt ggaacaacga cgccctggcg cgtcgccaac tgtcggctcc ggcggtgttc    960
   tccagcaacg tggtggtcgg cgacctggaa ggctacgtgc acctgctgag ccaggtggac   1020
   ggtcgcttcg tcggtcgcga gcgggtcgac agcgatggcg tgcgggttcg tccgctggtg   1080
   gtcgggagct ggatgtacgt gttcggcaac ggtggcaagc tcgtcgccta caccatccgc   1140
   tag                                                                 1143
<210> 6
   <211> 380
   <212> PRT
   <213> Pseudomonas aeruginosa
<400> 6
   Met Val Gln Trp Lys His Ala Ala Leu Leu Ala Leu Ala Leu Ala Val
   1               5                   10                  15
   Val Gly Cys Ser Ser Asn Ser Lys Lys Glu Leu Pro Pro Ala Glu Leu
               20                  25                  30
   Thr Asp Phe Lys Glu Glu Val Val Leu Ser Lys Gln Trp Ser Arg Ser
           35                  40                  45
   Val Gly Asp Gly Gln Gly Asp Leu Tyr Asn Leu Leu Glu Pro Ala Val
       50                  55                  60
   Asp Gly Ser Thr Ile Tyr Ala Ala Ser Ala Glu Gly Arg Val Met Ala
   65                  70                  75                  80
   Ile Gln Arg Glu Thr Gly Asp Val Leu Trp Lys Lys Asp Leu Glu Arg
                   85                  90                  95
   Pro Val Ser Gly Gly Val Gly Val Gly Tyr Gly Leu Val Leu Val Gly
               100                 105                 110
   Thr Leu Arg Gly Asp Val Ile Ala Leu Asp Glu Ala Thr Gly Lys Lys
   115 120 125
   Lys Trp Thr Lys Arg Val Asn Ser Glu Val Leu Ser Ala Pro Ala Thr
   130 135 140
   Asn Gly Asp Val Val Val Val Gln Thr Gln Asp Asp Lys Leu Ile Gly
   145 150 155 160
   Leu Asp Ala Ala Ser Gly Asp Gln Arg Trp Ile Tyr Glu Ser Thr Val
   165 170 175
   Pro Val Leu Thr Leu Arg Gly Thr Gly Ala Pro Leu Ile Ala Gly Asn
   180 185 190
   Met Ala Leu Ala Gly Leu Ala Ser Gly Lys Val Val Ala Val Asp Val
   195 200 205
   Gln Arg Gly Leu Pro Ile Trp Glu Gln Arg Val Ala Ile Pro Gln Gly
   210 215 220
   Arg Ser Glu Leu Asp Arg Val Val Asp Ile Asp Gly Gly Leu Leu Leu
   225 230 235 240
   Ser Gly Asp Thr Leu Tyr Val Val Ser Tyr Gln Gly Arg Ala Ala Ala
   245 250 255
   Leu Asp Val Asn Ser Gly Arg Leu Leu Trp Gln Arg Glu Ala Ser Ser
   260 265 270
   Tyr Val Gly Val Ala Glu Gly Phe Gly Asn Ile Tyr Val Ser Gln Ala
   275 280 285
   Ser Gly Ser Val Glu Gly Leu Asp Ser Arg Gly Ala Ser Ser Leu Trp
   290 295 300
   Asn Asn Asp Ala Leu Ala Arg Arg Gln Leu Ser Ala Pro Ala Val Phe
   305 310 315 320
   Ser Ser Asn Val Val Val Gly Asp Leu Glu Gly Tyr Val His Leu Leu
   325 330 335
   Ser Gln Val Asp Gly Arg Phe Val Gly Arg Glu Arg Val Asp Ser Asp
   340 345 350
   Gly Val Arg Val Arg Pro Leu Val Val Gly Ser Trp Met Tyr Val Phe
   355 360 365
   Gly Asn Gly Gly Lys Leu Val Ala Tyr Thr Ile Arg
   370 375 380
<210> 7
   <211> 36
   <212> DNA
   <213> Artificial Sequence
<220>
   <223> primer sequence
<400> 7
   gggaattcca tatggcgcag aagaatccga cagtcg 36
<210> 8
   <211> 33
   <212> DNA
   <213> Artificial Sequence
<220>
   <223> primer sequence
<400> 8
   ataagaatgc ggccgctggc gtccgcaggc ggt 33
<210> 9
   <211> 30
   <212> DNA
   <213> Artificial Sequence
<220>
   <223> primer sequence
<400> 9
   gggcagatct tgatggcgca gaagaatccg 30
<210> 10
   <211> 30
   <212> DNA
   <213> Artificial Sequence
<220>
   <223> primer sequence
<400> 10
   gatcctctag attggcgtcc gcaggcggtc 30
<210> 11
   <211> 31
   <212> DNA
   <213> Artificial Sequence
<220>
   <223> primer sequence
<400> 11
   gggaattcca tatgggcttc caactgcgcg g 31
<210> 12
   <211> 28
   <212> DNA
   <213> Artificial Sequence
<220>
   <223> primer sequence
<400> 12
   cgccaagctt cggggtgggg aactcgat 28
<210> 13
   <211> 30
   <212> DNA
   <213> Artificial Sequence
<220>
   <223> primer sequence
<400> 13
   cgaggatcct atgtgcggct tccaactgcg 30
<210> 14
   <211> 31
   <212> DNA
   <213> Artificial Sequence
<220>
   <223> primer sequence
<400> 14
   cagaagcttc ggggtgggga actcgatcgg c 31
<210> 15
   <211> 38
   <212> DNA
   <213> Artificial Sequence
<220>
   <223> primer sequence
<400> 15
   gggaattcca tatgagcagc aacagcaaga aggaactc 38
<210> 16
   <211> 28
   <212> DNA
   <213> Artificial Sequence
<220>
   <223> primer sequence
<400> 16
   cgccaagctt gcggatggtg taggcgac 28
<210> 17
   <211> 30
   <212> DNA
   <213> Artificial sequence
<220>
   <223> primer sequence
<400> 17
   cgaggatcct atgagcaaga aggaactccc 30
<210> 18
   <211> 31
   <212> DNA
   <213> Artificial Sequence
<220>
   <223> primer sequence
<400> 18
   cagaagcttc tagcggattg gtgtaggcga c 31
<210> 19
   <211> 1197
   <212> DNA
   <213> Pseudomonas aeruginosa
<400> 19
   atgcgcagcc ttcttctctc ctcgctggcc ctgctacccg ccctggccct ggcgcaaccc 60
   gacgcctcga gcttcccttc ctgcctcgcc ggcctgcaga agaaggccca ggcgcagggc 120
   atttccgccg acagttatga gcgcttcacc agcggcctgc aggccgacct cagcgtgctc 180
   gacctgctcg acgcgcagcc ggagttcacc accccgctgt gggactacct ggccggcctg 240
   gtggacgagc agcgggtcag cgatggcaag gcgatgctcg cccagcacga caagctgctc 300
   gaccaggtgg ccgcgcgcta cggcgtggac aagtacacgg tggtggcggt gtggggcgtg 360
   gaaagcgact acgggcggat cttcggcaag cgtccgctgc tgacctcgct gtcgaccctg 420
   tcctgctacg ggcgccgcca gtcgttcttc cagggcgagt tcctcgccac cctgaagctg 480
   ttgcaggccg gcgacatccg cgacgccggc atcaccggct cctgggccgg ggccttcggc 540
   catacccagt tcatgccatc gacctacgcg cggatcgccg tggacttcga cggcgacggt 600
   cgccgcgacc tggtaggcag cgtgccggat gccctcggtt ccaccgccaa ctacctgaag 660
   aaggctggct ggcgcacggg acagccgtgg ggctatgaag tgaaggtgcc ggccgacttc 720
   cccgccagcc tggccgggcg cggcaagcgc cagccgctgt cggcctgggt cgcccgtggg 780
   gtgaggcggg tcgacggcca gccgctgccg ggcggcgacg agaaggccgc gatcctcctg 840
   ccggccgggg cccagggccc ggccttcctg gtctatcgca actacgatgc gatctattcc 900
   tacaacgccg cggaaagcta cgcgctggcc atcgccctgc tttccgaccg cctgcgcggc 960
   ggcagcggcc tggtggcgtc ctggccgacc gacgacccgg gcatcagccg gctcgagcgc 1020
   aagcaattgc agaaggcgtt gctggcgcgc ggctacgaca tcggcgaggc cgacgggctg 1080
   atcggcacca gcacgcgcaa ggcgatccag gccgagcaga agcgcctcgg cctgaccccg 1140
   gccgacggtc gcgccgggcg caagatcctc gaggcgctga agggcgccca gccctga 1197
<210> 20
   <211> 398
   <212> PRT
   <213> Pseudomonas aeruginosa
<400> 20
   Met Arg Ser Leu Leu Leu Ser Ser Leu Ala Leu Leu Pro Ala Leu Ala
   1               5                   10                  15
   Leu Ala Gln Pro Asp Ala Ser Ser Phe Pro Ser Cys Leu Ala Gly Leu
               20                  25                  30
   Gln Lys Lys Ala Gln Ala Gln Gly Ile Ser Ala Asp Ser Tyr Glu Arg
           35                  40                  45
   Phe Thr Ser Gly Leu Gln Ala Asp Leu Ser Val Leu Asp Leu Leu Asp
       50                  55                  60
   Ala Gln Pro Glu Phe Thr Thr Pro Leu Trp Asp Tyr Leu Ala Gly Leu
   65                  70                  75                  80
   Val Asp Glu Gln Arg Val Ser Asp Gly Lys Ala Met Leu Ala Gln His
                   85                  90                  95
   Asp Lys Leu Leu Asp Gln Val Ala Ala Arg Tyr Gly Val Asp Lys Tyr
               100                 105                 110
   Thr Val Val Ala Val Trp Gly Val Glu Ser Asp Tyr Gly Arg Ile Phe
           115                 120                 125
   Gly Lys Arg Pro Leu Leu Thr Ser Leu Ser Thr Leu Ser Cys Tyr Gly
       130                 135                 140
   Arg Arg Gln Ser Phe Phe Gln Gly Glu Phe Leu Ala Thr Leu Lys Leu
   145                 150                 155                 160
   Leu Gln Ala Gly Asp Ile Arg Asp Ala Gly Ile Thr Gly Ser Trp Ala
                   165                 170                 175
   Gly Ala Phe Gly His Thr Gln Phe Met Pro Ser Thr Tyr Ala Arg Ile
               180                 185                 190
   Ala Val Asp Phe Asp Gly Asp Gly Arg Arg Asp Leu Val Gly Ser Val
           195                 200                 205
   Pro Asp Ala Leu Gly Ser Thr Ala Asn Tyr Leu Lys Lys Ala Gly Trp
       210                 215                 220
   Arg Thr Gly Gln Pro Trp Gly Tyr Glu Val Lys Val Pro Ala Asp Phe
   225                 230                 235                 240
   Pro Ala Ser Leu Ala Gly Arg Gly Lys Arg Gln Pro Leu Ser Ala Trp
   245 250 255
   Val Ala Arg Gly Val Arg Arg Val Asp Gly Gln Pro Leu Pro Gly Gly
   260 265 270
   Asp Glu Lys Ala Ala Ile Leu Leu Pro Ala Gly Ala Gln Gly Pro Ala
   275 280 285
   Phe Leu Val Tyr Arg Asn Tyr Asp Ala Ile Tyr Ser Tyr Asn Ala Ala
   290 295 300
   Glu Ser Tyr Ala Leu Ala Ile Ala Leu Leu Ser Asp Arg Leu Arg Gly
   305 310 315 320
   Gly Ser Gly Leu Val Ala Ser Trp Pro Thr Asp Asp Pro Gly Ile Ser
   325 330 335
   Arg Leu Glu Arg Lys Gln Leu Gln Lys Ala Leu Leu Ala Arg Gly Tyr
   340 345 350
   Asp Ile Gly Glu Ala Asp Gly Leu Ile Gly Thr Ser Thr Arg Lys Ala
   355 360 365
   Ile Gln Ala Glu Gln Lys Arg Leu Gly Leu Thr Pro Ala Asp Gly Arg
   370 375 380
   Ala Gly Arg Lys Ile Leu Glu Ala Leu Lys Gly Ala Gln Pro
   385 390 395
<210> 21
   <211> 1023
   <212> DNA
   <213> Pseudomonas aeruginosa
<400> 21
   gtgaagaacg caatgcaagt actgcgtaca tgggcggcca ggggcgtcca atgggtcggc 60
   gtagccggcg tcattggcct gtccggggcg gcccaggcgg gggactacga cggctcgccg 120
   caagtggccg agttcgtcag cgaaatgacc cgcgactacg gcttcgccgg agagcagctg 180
   atggggctgt tccgcgacgt gaaccgcaag cagtcgatcc tcgatgcgat ctcgcgcccg 240
   gccgagcggg tcaagcagtg gaaggaatac cggccgatct tcatcagcga cgcgcgcatc 300
   agtcgtggcg tcgacttctg gaacaagcat gccgaagacc tggcgcgggc ggagaaggaa 360
   tacggcgtgc cggccgagat catcgtctcg atcatcggcg tggaaacctt cttcggccgc 420
   aacaccggca gttaccgggt gatggacgcg ctgtccaccc tcggcttcga ctacccgccg 480
   cgggccgact tcttccgcaa ggagttgcgc gagttcctcc tgctcgcccg cgaacagcag 540
   gtcgacccgc tcagcctgac cggctcctac gccggcgcca tgggcctgcc acaattcatg 600
   ccgagcagct tccgcgccta cgcggtggac ttcgacggcg atggccacat caatatctgg 660
   agcgacccga ccgatgccat cggtagcgtc gccagctact tcaagcagca cggctgggtc 720
   accggcgagc cggtggtctc ggtggccgag atcaacgacg agagcgccga gagcgcggtg 780
   accaggggcg tcgacccgac catgagcctg ggcgagctgc gtgcccgcgg ctggcgcacc 840
   cacgatgcgc tgcgcgacga ccagaaggtc acggcgatgc gtttcgtcgg cgacaagggc 900
   atcgagtatt gggtcggttt gccgaacttc tacgtgatca cccgctataa tcgcagcgcc 960
   atgtatgcca tggcggttta tcagctggcg ggcgagattg cccgcgcgcg aggtgcccat 1020
   tga 1023
<210> 22
   <211> 340
   <212> PRT
   <213> Pseudomonas aeruginosa
<400> 22
   Met Lys Asn Ala Met Gln Val Leu Arg Thr Trp Ala Ala Arg Gly Val
   1 5 10 15
   Gln Trp Val Gly Val Ala Gly Val Ile Gly Leu Ser Gly Ala Ala Gln
   20 25 30
   Ala Gly Asp Tyr Asp Gly Ser Pro Gln Val Ala Glu Phe Val Ser Glu
   35 40 45
   Met Thr Arg Asp Tyr Gly Phe Ala Gly Glu Gln Leu Met Gly Leu Phe
   50 55 60
   Arg Asp Val Asn Arg Lys Gln Ser Ile Leu Asp Ala Ile Ser Arg Pro
   65 70 75 80
   Ala Glu Arg Val Lys Gln Trp Lys Glu Tyr Arg Pro Ile Phe Ile Ser
   85 90 95
   Asp Ala Arg Ile Ser Arg Gly Val Asp Phe Trp Asn Lys His Ala Glu
   100 105 110
   Asp Leu Ala Arg Ala Glu Lys Glu Tyr Gly Val Pro Ala Glu Ile Ile
   115 120 125
   Val Ser Ile Ile Gly Val Glu Thr Phe Phe Gly Arg Asn Thr Gly Ser
   130 135 140
   Tyr Arg Val Met Asp Ala Leu Ser Thr Leu Gly Phe Asp Tyr Pro Pro
   145 150 155 160
   Arg Ala Asp Phe Phe Arg Lys Glu Leu Arg Glu Phe Leu Leu Leu Ala
   165 170 175
   Arg Glu Gln Gln Val Asp Pro Leu Ser Leu Thr Gly Ser Tyr Ala Gly
   180 185 190
   Ala Met Gly Leu Pro Gln Phe Met Pro Ser Ser Phe Arg Ala Tyr Ala
   195 200 205
   Val Asp Phe Asp Gly Asp Gly His Ile Asn Ile Trp Ser Asp Pro Thr
   210 215 220
   Asp Ala Ile Gly Ser Val Ala Ser Tyr Phe Lys Gln His Gly Trp Val
   225 230 235 240
   Thr Gly Glu Pro Val Val Ser Val Ala Glu Ile Asn Asp Glu Ser Ala
   245 250 255
   Glu Ser Ala Val Thr Arg Gly Val Asp Pro Thr Met Ser Leu Gly Glu
   260 265 270
   Leu Arg Ala Arg Gly Trp Arg Thr His Asp Ala Leu Arg Asp Asp Gln
   275 280 285
   Lys Val Thr Ala Met Arg Phe Val Gly Asp Lys Gly Ile Glu Tyr Trp
   290 295 300
   Val Gly Leu Pro Asn Phe Tyr Val Ile Thr Arg Tyr Asn Arg Ser Ala
   305 310 315 320
   Met Tyr Ala Met Ala Val Tyr Gln Leu Ala Gly Glu Ile Ala Arg Ala
   325 330 335
   Arg Gly Ala His
   340
<210> 23
   <211> 1104
   <212> DNA
   <213> Pseudomonas aeruginosa
<400> 23
   atgcgccgta ccgccctcgc cctgcccctg ttccttctgg tctcagcatg cagcagcgaa 60
   ccgacgccac caccgaaacc cgccgccaaa ccccaggccc gcaccgtcat ttcaccccgc 120
   cccgtacgcc agtcggtgca accgatactg ccgctgcgcg gcgattacgc gaacaatccg 180
   gcggcacagc acttcatcga caggatggtc agccagcacg gcttcaaccg ccagcaactg 240
   cacgatctgt tcgcccagac ccagcgcctg gactgggtga tccgcctgat ggaccggcaa 300
   gccccgacct ataccccacc cagcggaccg aacggcgcct ggctgcgcta ccggaagaag 360
   ttcgtcacgc caggcaacgt acagaacggc gtgctgttct gggaccaata cgaaaccgac 420
   ctgcaacggg catcgcgcgt ctacggcgtg ccgccggaga tcatcgtcgg catcatcggc 480
   gtggaaaccc gctgggggcg tgtgatgggc aagacgcgga tcatcgatgc gctgtccacc 540
   ctgtccttct cctaccctcg ccgcgcggaa ttcttcagcg gcgaactgga gcaattcctc 600
   ctccaggcgc gcaaggaagg caccgacccg ctggccctgc gcggttccta tgccggcgcc 660
   atgggctacg gccagttcat gccgtcttca ttcaccaagt acgcggtgga cttcgatggc 720
   gatgggcata tcgacctgtg gaatccgcgt gacgccatcg gcagcgtcgc caactatttc 780
   aagcagcacg gctgggtcag cggcgatcgc gtggcggttc ccgccagtgg ccgggctccc 840
   tcgctggaag atggcttcaa gacgctgtac ccgctggacg tgctcgcttc cgccggatta 900
   cgcccgcagg gtccgctcgg cggccaccgg caagccagcc tgctgcgcct ggacatgggc 960
   aggaactacc agtactggta cggcctgccg aacttctacg tgatcacccg ctataaccac 1020
   agcacccact acgcgatggc cgtctgggaa ctgggcaagg aagtcgaccg ggtgcgtcac 1080
   cgctccgtcg tcaggcagga ttag 1104
<210> 24
   <211> 367
   <212> PRT
   <213> Pseudomonas aeruginosa
<400> 24
   Met Arg Arg Thr Ala Leu Ala Leu Pro Leu Phe Leu Leu Val Ser Ala
   1 5 10 15
   Cys Ser Ser Glu Pro Thr Pro Pro Pro Lys Pro Ala Ala Lys Pro Gln
   20 25 30
   Ala Arg Thr Val Ile Ser Pro Arg Pro Val Arg Gln Ser Val Gln Pro
   35 40 45
   Ile Leu Pro Leu Arg Gly Asp Tyr Ala Asn Asn Pro Ala Ala Gln His
   50 55 60
   Phe Ile Asp Arg Met Val Ser Gln His Gly Phe Asn Arg Gln Gln Leu
   65 70 75 80
   His Asp Leu Phe Ala Gln Thr Gln Arg Leu Asp Trp Val Ile Arg Leu
   85 90 95
   Met Asp Arg Gln Ala Pro Thr Tyr Thr Pro Pro Ser Gly Pro Asn Gly
               100                 105                 110
   Ala Trp Leu Arg Tyr Arg Lys Lys Phe Val Thr Pro Gly Asn Val Gln
           115                 120                 125
   Asn Gly Val Leu Phe Trp Asp Gln Tyr Glu Thr Asp Leu Gln Arg Ala
       130                 135                 140
   Ser Arg Val Tyr Gly Val Pro Pro Glu Ile Ile Val Gly Ile Ile Gly
   145                 150                 155                 160
   Val Glu Thr Arg Trp Gly Arg Val Met Gly Lys Thr Arg Ile Ile Asp
                   165                 170                 175
   Ala Leu Ser Thr Leu Ser Phe Ser Tyr Pro Arg Arg Ala Glu Phe Phe
               180                 185                 190
   Ser Gly Glu Leu Glu Gln Phe Leu Leu Gln Ala Arg Lys Glu Gly Thr
           195                 200                 205
   Asp Pro Leu Ala Leu Arg Gly Ser Tyr Ala Gly Ala Met Gly Tyr Gly
       210                 215                 220
   Gln Phe Met Pro Ser Ser Phe Thr Lys Tyr Ala Val Asp Phe Asp Gly
   225                 230                 235                 240
   Asp Gly His Ile Asp Leu Trp Asn Pro Arg Asp Ala Ile Gly Ser Val
                   245                 250                 255
   Ala Asn Tyr Phe Lys Gln His Gly Trp Val Ser Gly Asp Arg Val Ala
               260                 265                 270
   Val Pro Ala Ser Gly Arg Ala Pro Ser Leu Glu Asp Gly Phe Lys Thr
           275                 280                 285
   Leu Tyr Pro Leu Asp Val Leu Ala Ser Ala Gly Leu Arg Pro Gln Gly
       290                 295                 300
   Pro Leu Gly Gly His Arg Gln Ala Ser Leu Leu Arg Leu Asp Met Gly
   305                 310                 315                 320
   Arg Asn Tyr Gln Tyr Trp Tyr Gly Leu Pro Asn Phe Tyr Val Ile Thr
                   325                 330                 335
   Arg Tyr Asn His Ser Thr His Tyr Ala Met Ala Val Trp Glu Leu Gly
               340                 345                 350
   Lys Glu Val Asp Arg Val Arg His Arg Ser Val Val Arg Gln Asp
           355                 360                 365
<210> 25
   <211> 42
   <212> DNA
   <213> Artificial sequence
<220>
   <223> primer sequence
<400> 25
   gagttccata tgagcttccc ttcctgcctc gccggcctgc ag 42
<210> 26
   <211> 41
   <212> DNA
   <213> Artificial Sequence
<220>
   <223> primer sequence
<400> 26
   cgctgaggat cctcacttct gcaattgctt gcgctcgagc c 41
<210> 27
   <211> 32
   <212> DNA
   <213> Artificial Sequence
<220>
   <223> primer sequence
<400> 27
   gggaattcca tatgggggcg gcccaggcgg cg 32
<210> 28
   <211> 30
   <212> DNA
   <213> Artificial Sequence
<220>
   <223> primer sequence
<400> 28
   gcgctgagga tcctcaatgg gcacctcgcg 30
<210> 29
   <211> 30
   <212> DNA
   <213> Artificial sequence
<220>
   <223> primer sequence
<400> 29
   gggaattcca tatgagcagc gaaccgacgc 30
<210> 30
   <211> 30
   <212> DNA
   <213> Artificial sequence
<220>
   <223> primer sequence
<400> 30 .
   cgccaagctt ctaatcctgc ctgacgacgg 30
<210> 31
   <211> 419
   <212> PRT
   <213> Pseudomonas aeruginosa
<400> 31
   Ala Cys Ala Gln Lys Asn Pro Thr Val Glu Tyr Asn Gln Pro Ala Ala
   1 5 10 15
   Pro Leu Gln Thr Lys Ala Pro Phe Ser Gly Ala Gly Pro Ala Ala Ser
   20 25 30
   Val Pro Ala Gly Ala Pro Asn Glu Ala Gln Pro Gly Gln Ser Phe Glu
   35 40 45
   Gln Trp Arg Asp Ala Phe Arg Gln Gln Ala Leu Ala Gly Gly Ile Asp
   50 55 60
   Ala Gln Thr Phe Asp Arg Ala Phe Ala Gly Val Gln Pro Asp Pro Ala
   65 70 75 80
   Val Val Glu Ala Asp Arg Ser Gln Pro Glu Phe Thr Arg Pro Val Trp
   85 90 95
   Lys Tyr Leu Glu Gly Ala Leu Asp Pro Leu Arg Val Arg Gln Gly Gln
   100 105 110
   Ala Arg Leu Ala Gln His Ala Arg Ile Leu Gly Glu Val Asp Ala Arg
   115 120 125
   Tyr Ala Val Asp Ala Asp Ala Val Val Ala Ile Trp Gly Met Glu Ser
   130 135 140
   Asn Tyr Gly Ser His Met Gly Asn Lys Asn Val Ile Arg Ser Leu Ala
   145 150 155 160
   Thr Leu Ala Tyr Glu Gly Arg Arg Pro Glu Phe Ala His Ala Gln Leu
   165 170 175
   Leu Ala Ala Leu Lys Ile Leu Gln His Gly Asp Val Pro Ala Ser Phe
   180 185 190
   Met Ile Gly Ser Trp Ala Gly Ala Met Gly Gln Thr Gln Phe Ile Pro
   195 200 205
   Thr Thr His Asn Gln Tyr Ala Val Asp Phe Asp Gly Asp Gly Lys Arg
   210 215 220
   Asp Ile Trp Gly Ser Pro Gly Asp Ala Leu Ala Ser Thr Ala Asn Tyr
   225 230 235 240
   Leu Lys Ala Ser Gly Trp Ile Ala Gly Gln Pro Trp Gly Phe Glu Val
   245 250 255
   Arg Leu Pro Ala Gly Phe Asp Tyr Ser Leu Ala Glu Leu Thr Ile Arg
   260 265 270
   Lys Pro Leu Gly Glu Trp Gln Gly Met Gly Val Gln Gly Val Asn Gly
   275 280 285
   Gly Pro Leu Pro Ser Gly Leu Ser Gly Glu Gln Ala Ser Leu Leu Leu
   290 295 300
   Pro Ala Gly His Arg Gly Pro Ala Phe Leu Val Leu His Asn Phe Arg
   305 310 315 320
   Ala Ile Leu Lys Tyr Asn Asn Ser Ser Ala Tyr Ala Leu Ala Val Gly
   325 330 335
   Leu Leu Ala Asp Ser Phe Lys Gly Gly Gly Arg Ile Val Gly Ala Trp
   340 345 350
   Pro Leu Glu Asp Val Pro Leu Ser Arg Ser Gln Arg Ile Glu Leu Gln
   355 360 365
   Arg Gln Leu Ala Ala Arg Gly His Asp Pro Gly Ala Val Asp Gly Ile
   370 375 380
   Ile Gly Ala Asn Thr Arg Lys Ala Ile Arg Ala Cys Gln Gln Glu Phe
   385 390 395 400
   Gly Trp Pro Ala Asp Gly Tyr Pro Thr Pro Ala Leu Leu Asp Arg Leu
   405 410 415
   Arg Thr Pro
<210> 32
   <211> 398
   <212> PRT
   <213> Pseudomonas aeruginosa
<400> 32
   Met Arg Ser Leu Leu Leu Ser Ser Leu Ala Leu Leu Pro Ala Leu Ala
   1 5 10 15
   Leu Ala Gln Pro Asp Ala Ser Ser Phe Pro Ser Cys Leu Ala Gly Leu
   20 25 30
   Gln Lys Lys Ala Gln Ala Gln Gly Ile Ser Ala Asp Ser Tyr Glu Arg
   35 40 45
   Phe Thr Ser Gly Leu Gln Ala Asp Leu Ser Val Leu Asp Leu Leu Asp
   50 55 60
   Ala Gln Pro Glu Phe Thr Thr Pro Leu Trp Asp Tyr Leu Ala Gly Leu
   65 70 75 80
   Val Asp Glu Gln Arg Val Ser Asp Gly Lys Ala Met Leu Ala Gln His
   85 90 95
   Asp Lys Leu Leu Asp Gln Val Ala Ala Arg Tyr Gly Val Asp Lys Tyr
   100 105 110
   Thr Val Val Ala Val Trp Gly Val Glu Ser Asp Tyr Gly Arg Ile Phe
   115 120 125
   Gly Lys Arg Pro Leu Leu Thr Ser Leu Ser Thr Leu Ser Cys Tyr Gly
       130                 135                 140
   Arg Arg Gln Ser Phe Phe Gln Gly Glu Phe Leu Ala Thr Leu Lys Leu
   145                 150                 155                 160
   Leu Gln Ala Gly Asp Ile Arg Asp Ala Gly Ile Thr Gly Ser Trp Ala
                   165                 170                 175
   Gly Ala Phe Gly His Thr Gln Phe Met Pro Ser Thr Tyr Ala Arg Ile
               180                 185                 190
   Ala Val Asp Phe Asp Gly Asp Gly Arg Arg Asp Leu Val Gly Ser Val
           195                 200                 205
   Pro Asp Ala Leu Gly Ser Thr Ala Asn Tyr Leu Lys Lys Ala Gly Trp
       210                 215                 220
   Arg Thr Gly Gln Pro Trp Gly Tyr Glu Val Lys Val Pro Ala Asp Phe
   225                 230                 235                 240
   Pro Ala Ser Leu Ala Gly Arg Gly Lys Arg Gln Pro Leu Ser Ala Trp
                   245                 250                 255
   Val Ala Arg Gly Val Arg Arg Val Asp Gly Gln Pro Leu Pro Gly Gly
               260                 265                 270
   Asp Glu Lys Ala Ala Ile Leu Leu Pro Ala Gly Ala Gln Gly Pro Ala
           275                 280                 285
   Phe Leu Val Tyr Arg Asn Tyr Asp Ala Ile Tyr Ser Tyr Asn Ala Ala
       290                 295                 300
   Glu Ser Tyr Ala Leu Ala Ile Ala Leu Leu Ser Asp Arg Leu Arg Gly
   305                 310                 315                 320
   Gly Ser Gly Leu Val Ala Ser Trp Pro Thr Asp Asp Pro Gly Ile Ser
                   325                 330                 335
   Arg Leu Glu Arg Lys Gln Leu Gln Lys Ala Leu Leu Ala Arg Gly Tyr
               340                 345                 350
   Asp Ile Gly Glu Ala Asp Gly Leu Ile Gly Thr Ser Thr Arg Lys Ala
           355                 360                 365
   Ile Gln Ala Glu Gln Lys Arg Leu Gly Leu Thr Pro Ala Asp Gly Arg
       370                 375                 380
   Ala Gly Arg Lys Ile Leu Glu Ala Leu Lys Gly Ala Gln Pro
   385                 390                 395
<210> 33
   <211> 348
   <212> PRT
   <213> Pseudomonas aeruginosa
<400> 33
   Val Pro Ala Gly Ala Pro Asn Glu Ala Gln Pro Gly Gln Ser Phe Glu
   1               5                   10                  15
   Gln Trp Arg Asp Ala Phe Arg Gln Gln Ala Leu Ala Gly Gly Ile Asp
               20                  25                  30
   Ala Gln Thr Phe Asp Arg Ala Phe Ala Gly Val Gln Pro Asp Pro Ala
           35                  40                  45
   Val Val Glu Ala Asp Arg Ser Gln Pro Glu Phe Thr Arg Pro Val Trp
       50                  55                  60
   Lys Tyr Leu Glu Gly Ala Leu Asp Pro Leu Arg Val Arg Gln Gly Gln
   65                  70                  75                  80
   Ala Arg Leu Ala Gln His Ala Arg Ile Leu Gly Glu Val Asp Ala Arg
                   85                  90                  95
   Tyr Ala Val Asp Ala Asp Ala Val Val Ala Ile Trp Gly Met Glu Ser
               100                 105                 110
   Asn Tyr Gly Ser His Met Gly Asn Lys Asn Val Ile Arg Ser Leu Ala
           115                 120                 125
   Thr Leu Ala Tyr Glu Gly Arg Arg Pro Glu Phe Ala His Ala Gln Leu
       130                 135                 140
   Leu Ala Ala Leu Lys Ile Leu Gln His Gly Asp Val Pro Ala Ser Phe
   145                 150                 155                 160
   Met Ile Gly Ser Trp Ala Gly Ala Met Gly Gln Thr Gln Phe Ile Pro
                   165                 170                 175
   Thr Thr His Asn Gln Tyr Ala Val Asp Phe Asp Gly Asp Gly Lys Arg
               180                 185                 190
   Asp Ile Trp Gly Ser Pro Gly Asp Ala Leu Ala Ser Thr Ala Asn Tyr
           195                 200                 205
   Leu Lys Ala Ser Gly Trp Ile Ala Gly Gln Pro Trp Gly Phe Glu Val
       210                 215                 220
   Arg Leu Pro Ala Gly Phe Asp Tyr Ser Leu Ala Glu Leu Thr Ile Arg
   225 230 235 240
   Lys Pro Leu Gly Glu Trp Gln Gly Met Gly Val Gln Gly Val Asn Gly
   245 250 255
   Gly Pro Leu Pro Ser Gly Leu Ser Gly Glu Gln Ala Ser Leu Leu Leu
   260 265 270
   Pro Ala Gly His Arg Gly Pro Ala Phe Leu Val Leu His Asn Phe Arg
   275 280 285
   Ala Ile Leu Lys Tyr Asn Asn Ser Ser Ala Tyr Ala Leu Ala Val Gly
   290 295 300
   Leu Leu Ala Asp Ser Phe Lys Gly Gly Gly Arg Ile Val Gly Ala Trp
   305 310 315 320
   Pro Leu Glu Asp Val Pro Leu Ser Arg Ser Gln Arg Ile Glu Leu Gln
   325 330 335
   Arg Gln Leu Ala Ala Arg Gly His Asp Pro Gly Ala
   340 345
<210> 34
   <211> 325
   <212> PRT
   <213> Pseudomonas aeruginosa
<400> 34
   Val Gln Trp Val Gly Val Ala Gly Val Ile Gly Leu Ser Gly Ala Ala
   1 5 10 15
   Gln Ala Gly Asp Tyr Asp Gly Ser Pro Gln Val Ala Glu Phe Val Ser
   20 25 30
   Glu Met Thr Arg Asp Tyr Gly Phe Ala Gly Glu Gln Leu Met Gly Leu
   35 40 45
   Phe Arg Asp Val Asn Arg Lys Gln Ser Ile Leu Asp Ala Ile Ser Arg
   50 55 60
   Pro Ala Glu Arg Val Lys Gln Trp Lys Glu Tyr Arg Pro Ile Phe Ile
   65 70 75 80
   Ser Asp Ala Arg Ile Ser Arg Gly Val Asp Phe Trp Asn Lys His Ala
   85 90 95
   Glu Asp Leu Ala Arg Ala Glu Lys Glu Tyr Gly Val Pro Ala Glu Ile
   100 105 110
   Ile Val Ser Ile Ile Gly Val Glu Thr Phe Phe Gly Arg Asn Thr Gly
   115 120 125
   Ser Tyr Arg Val Met Asp Ala Leu Ser Thr Leu Gly Phe Asp Tyr Pro
   130 135 140
   Pro Arg Ala Asp Phe Phe Arg Lys Glu Leu Arg Glu Phe Leu Leu Leu
   145 150 155 160
   Ala Arg Glu Gln Gln Val Asp Pro Leu Ser Leu Thr Gly Ser Tyr Ala
   165 170 175
   Gly Ala Met Gly Leu Pro Gln Phe Met Pro Ser Ser Phe Arg Ala Tyr
   180 185 190
   Ala Val Asp Phe Asp Gly Asp Gly His Ile Asn Ile Trp Ser Asp Pro
   195 200 205
   Thr Asp Ala Ile Gly Ser Val Ala Ser Tyr Phe Lys Gln His Gly Trp
   210 215 220
   Val Thr Gly Glu Pro Val Val Ser Val Ala Glu Ile Asn Asp Glu Ser
   225 230 235 240
   Ala Glu Ser Ala Val Thr Arg Gly Val Asp Pro Thr Met Ser Leu Gly
   245 250 255
   Glu Leu Arg Ala Arg Gly Trp Arg Thr His Asp Ala Leu Arg Asp Asp
   260 265 270
   Gln Lys Val Thr Ala Met Arg Phe Val Gly Asp Lys Gly Ile Glu Tyr
   275 280 285
   Trp Val Gly Leu Pro Asn Phe Tyr Val Ile Thr Arg Tyr Asn Arg Ser
   290 295 300
   Ala Met Tyr Ala Met Ala Val Tyr Gln Leu Ala Gly Glu Ile Ala Arg
   305 310 315 320
   Ala Arg Gly Ala His
   325
<210> 35
   <211> 405
   <212> PRT
   <213> Pseudomonas aeruginosa
<400> 35
   Met Arg Asn Pro Glu Arg Ser Ala Leu Leu Lys Val Ser Gly Leu Leu
   1               5                   10                  15
   Gly Ser Thr Val Val Ala Met Gly Leu Gly Leu Ser Ser Ala Cys Ala
               20                  25                  30
   Gln Lys Asn Pro Thr Val Glu Tyr Asn Gln Pro Ala Ala Pro Leu Gln
           35                  40                  45
   Thr Lys Ala Pro Phe Ser Gly Ala Gly Pro Ala Ala Ser Val Pro Ala
       50                  55                  60
   Gly Ala Pro Asn Glu Ala Gln Pro Gly Gln Ser Phe Glu Gln Trp Arg
   65                  70                  75                  80
   Asp Ala Phe Arg Gln Gln Ala Leu Ala Gly Gly Ile Asp Ala Gln Thr
                   85                  90                  95
   Phe Asp Arg Ala Phe Ala Gly Val Gln Pro Asp Pro Ala Val Val Glu
               100                 105                 110
   Ala Asp Arg Ser Gln Pro Glu Phe Thr Arg Pro Val Trp Lys Tyr Leu
           115                 120                 125
   Glu Gly Ala Leu Asp Pro Leu Arg Val Arg Gln Gly Gln Ala Arg Leu
       130                 135                 140
   Ala Gln His Ala Arg Ile Leu Gly Glu Val Asp Ala Arg Tyr Ala Val
   145                 150                 155                 160
   Asp Ala Asp Ala Val Val Ala Ile Trp Gly Met Glu Ser Asn Tyr Gly
                   165                 170                 175
   Ser His Met Gly Asn Lys Asn Val Ile Arg Ser Leu Ala Thr Leu Ala
               180                 185                 190
   Tyr Glu Gly Arg Arg Pro Glu Phe Ala His Ala Gln Leu Leu Ala Ala
           195                 200                 205
   Leu Lys Ile Leu Gln His Gly Asp Val Pro Ala Ser Phe Met Ile Gly
       210                 215                 220
   Ser Trp Ala Gly Ala Met Gly Gln Thr Gln Phe Ile Pro Thr Thr His
   225                 230                 235                 240
   Asn Gln Tyr Ala Val Asp Phe Asp Gly Asp Gly Lys Arg Asp Ile Trp
                   245                 250                 255
   Gly Ser Pro Gly Asp Ala Leu Ala Ser Thr Ala Asn Tyr Leu Lys Ala
   260 265 270
   Ser Gly Trp Ile Ala Gly Gln Pro Trp Gly Phe Glu Val Arg Leu Pro
   275 280 285
   Ala Gly Phe Asp Tyr Ser Leu Ala Glu Leu Thr Ile Arg Lys Pro Leu
   290 295 300
   Gly Glu Trp Gln Gly Met Gly Val Gln Gly Val Asn Gly Gly Pro Leu
   305 310 315 320
   Pro Ser Gly Leu Ser Gly Glu Gln Ala Ser Leu Leu Leu Pro Ala Gly
   325 330 335
   His Arg Gly Pro Ala Phe Leu Val Leu His Asn Phe Arg Ala Ile Leu
   340 345 350
   Lys Tyr Asn Asn Ser Ser Ala Tyr Ala Leu Ala Val Gly Leu Leu Ala
   355 360 365
   Asp Ser Phe Lys Gly Gly Gly Arg Ile Val Gly Ala Trp Pro Leu Glu
   370 375 380
   Asp Val Pro Leu Ser Arg Ser Gln Arg Ile Glu Leu Gln Arg Gln Leu
   385 390 395 400
   Ala Ala Arg Gly His
   405
<210> 36
   <211> 367
   <212> PRT
   <213> Pseudomonas aeruginosa
<400> 36
   Met Arg Arg Thr Ala Leu Ala Leu Pro Leu Phe Leu Leu Val Ser Ala
   1 5 10 15
   Cys Ser Ser Glu Pro Thr Pro Pro Pro Lys Pro Ala Ala Lys Pro Gln
   20 25 30
   Ala Arg Thr Val Ile Ser Pro Arg Pro Val Arg Gln Ser Val Gln Pro
   35 40 45
   Ile Leu Pro Leu Arg Gly Asp Tyr Ala Asn Asn Pro Ala Ala Gln His
   50 55 60
   Phe Ile Asp Arg Met Val Ser Gln His Gly Phe Asn Arg Gln Gln Leu
   65 70 75 80
   His Asp Leu Phe Ala Gln Thr Gln Arg Leu Asp Trp Val Ile Arg Leu
   85 90 95
   Met Asp Arg Gln Ala Pro Thr Tyr Thr Pro Pro Ser Gly Pro Asn Gly
   100 105 110
   Ala Trp Leu Arg Tyr Arg Lys Lys Phe Val Thr Pro Gly Asn Val Gln
   115 120 125
   Asn Gly Val Leu Phe Trp Asp Gln Tyr Glu Thr Asp Leu Gln Arg Ala
   130 135 140
   Ser Arg Val Tyr Gly Val Pro Pro Glu Ile Ile Val Gly Ile Ile Gly
   145 150 155 160
   Val Glu Thr Arg Trp Gly Arg Val Met Gly Lys Thr Arg Ile Ile Asp
   165 170 175
   Ala Leu Ser Thr Leu Ser Phe Ser Tyr Pro Arg Arg Ala Glu Phe Phe
   180 185 190
   Ser Gly Glu Leu Glu Gln Phe Leu Leu Gln Ala Arg Lys Glu Gly Thr
   195 200 205
   Asp Pro Leu Ala Leu Arg Gly Ser Tyr Ala Gly Ala Met Gly Tyr Gly
   210 215 220
   Gln Phe Met Pro Ser Ser Phe Thr Lys Tyr Ala Val Asp Phe Asp Gly
   225 230 235 240
   Asp Gly His Ile Asp Leu Trp Asn Pro Arg Asp Ala Ile Gly Ser Val
   245 250 255
   Ala Asn Tyr Phe Lys Gln His Gly Trp Val Ser Gly Asp Arg Val Ala
   260 265 270
   Val Pro Ala Ser Gly Arg Ala Pro Ser Leu Glu Asp Gly Phe Lys Thr
   275 280 285
   Leu Tyr Pro Leu Asp Val Leu Ala Ser Ala Gly Leu Arg Pro Gln Gly
   290 295 300
   Pro Leu Gly Gly His Arg Gln Ala Ser Leu Leu Arg Leu Asp Met Gly
   305 310 315 320
   Arg Asn Tyr Gln Tyr Trp Tyr Gly Leu Pro Asn Phe Tyr Val Ile Thr
   325 330 335
   Arg Tyr Asn His Ser Thr His Tyr Ala Met Ala Val Trp Glu Leu Gly
               340                 345                 350
   Lys Glu Val Asp Arg Val Arg His Arg Ser Val Val Arg Gln Asp
           355                 360                 365